# EUROPÄISCHE PATENTSCHRIFT

(11) **EP 1 432 313 B1**
(45) Veröffentlichungstag und Bekanntmachung des Hinweises auf die Patenterteilung: **23.01.2008**
(21) Anmeldenummer: 02762475.8
(22) Anmeldetag: 13.09.2002
(51) Int. Cl.: A01N 43/40, A01N 43/50, A01N 43/824, A01N 43/647, A61K 31/455, A61K 31/506, C07D 213/78, C07D 401/12, C07D 413/12, C07D 405/12, C07D 417/12, C07D 471/04, C07D 521/00, C07F 7/08, C07D 409/12

(54) **N-THIO-NICOTINAMID DERIVATE UND VERWANDTE VERBINDUNGEN ZUR VERWENDUNG ALS SCHÄDLINGSBEKÄMPFUNGSMITTEL**
N-THIO-NICOTINAMIDE DERIVATIVES AND RELATED COMPOUNDS FOR USE AS PESTICIDES
DERIVES DE N-THIO-NICOTINAMIDE ET COMPOSES APPARENTES ET LEUR UTILISATION COMME PESTICIDES

(30) Priorität: 24.09.2001 DE 10146873
(43) Veröffentlichungstag der Anmeldung: 30.06.2004
(73) Patentinhaber: Merial Limited, Duluth, GA 30096-4640 (US)
(72) Erfinder: BECKMANN, Marion, 65195 Wiesbaden (DE); ORT, Oswald, 61479 Glashütten (DE); DÖLLER, Uwe, 63110 Rodgau (DE); KRAUTSTRUNK, Gerhard, 61118 Bad Vilbel (DE); SCHAPER, Wolfgang, 86420 Diedorf (DE); LÜMMEN, Peter, 65510 Idstein (DE); JANS, Daniela, 61348 Bad Homburg v. d. H. (DE); HEMPEL, Waltraud, 65835 Liederbach (DE); WAIBEL, Jutta, Maria, 60529 Frankfurt (DE); KÜHN, Barbara, 65830 Kriftel (DE)
(74) Vertreter: Vossius & Partner
(86) Internationale Anmeldenummer: PCT/EP2002/010279
(87) Internationale Veröffentlichungsnummer: WO 2003/028458

(56) Entgegenhaltungen:
- EP-A- 0 580 374
- WO-A-01/09104
- WO-A-01/70692
- WO-A-99/16744
- US-A- 6 117 821
- PATENT ABSTRACTS OF JAPAN vol. 012, no. 033 (C-472), 30. Januar 1988 (1988-01-30) & JP 62 181261 A (NIPPON KAYAKU CO LTD (JP)), 8. August 1987 (1987-08-08)
- DATABASE WPI Section Ch, Week 200122 Derwent Publications Ltd., London, GB; Class C02, AN 2001-211706 XP002225174 & CN 1 274 528 A (SHENYANG INST CHEM ENG), 29. November 2000 (2000-11-29) -& DATABASE REGISTRY [Online] CHEMICAL ABSTRACTS SERVICE, COLUMBUS, OHIO, US; Database accession no. 342371-46-4 XP002228063

## Beschreibung

Die Erfindung betrifft heterocyclische Amid- und Iminderivate, Verfahren zu ihrer Herstellung, sie enthaltende Mittel sowie ihre Verwendung zur Bekämpfung von tierischen Schädlingen, insbesondere Arthropoden, wie Insekten und Acarina, und Helminthen.

Wegen des enormen Schadens, den Insekten beispielsweise durch Fraß an Nutzpflanzen, Lebensmittelvorräten, Holz und Textilien oder auch durch Krankheitsübertragung auf Mensch, Haustiere und Nutzpflanzen verursachen, ist die Verwendung von Insektiziden oder Repellentien nach wie vor unverzichtbar. Insektizide sind ein wichtiger Bestandteil der integrierten Schädlingskontrolle und tragen entscheidend zu Emteertrag und Kontinuität der Ernten in aller Welt bei.

Aus der US-A-6,028,101 sind substituierte carbocyclische und heterocyclische Verbindungen als Fungizide bekannt. Die allgemeine Formel der beschriebenen Verbindungen umfasst auch Pyridylamide, deren Amidstickstoff mit unterschiedlichen Gruppen, so auch Alkylthio substituiert sein kann. Eine konkrete Offenbarung für derartige Verbindungen findet sich allerdings nicht.

In der DE-A-36 00 288 wird die Verwendung von ausgewählten Amiden als Gegenmittel zur Verbesserung der Kulturpflanzen-Verträglichkeit von ausgewählten herbizid wirksamen Sulfonylharnstoff-Derivaten beschrieben. Als Amide lassen sich unter anderem Pyridincarbonsäurederivate einsetzen, die am Amidstickstoff mit einer Alkylthiogruppe substituiert sind. Eine konkrete Offenbarung für derartige Verbindungen findet sich allerdings nicht.

In der EP-A-434,097 wird ein Verfahren zur Entwicklung von silberhalogenidhaltigen Materialien für die Farbphotographie beschrieben. Dabei kommen unter anderem Entwicklermaterialien zum Einsatz, in denen Komponenten enthalten sind, die sich von heterocyclischen Iminderivaten ableiten. Eine konkrete Offenbarung für Verbindungen enthaltend Pyridylreste findet sich allerdings nicht.

Aus EP-A 0 580 374 sind Trifluormethylpyridinamide als Schädlingsbekämpfungsmittel bekannt.

Da sich aber die ökologischen und ökonomischen Anforderungen an moderne Insektizide laufend erhöhen, beispielsweise was Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Insektizide zu entwickeln, die zumindest in Teilbereichen Vorteile gegenüber den bekannten aufweisen.

Es wurde gefunden, daß Verbindungen der allgemeinen Formeln (I) und (II), gegebenenfalls auch als Salze, ein gutes Wirkungsspektrum gegenüber tierischen Schädlingen bei gleichzeitig guter Pflanzenverträglichkeit und günstigen toxikologischen Eigenschaften gegenüber Säugetieren und aquatischen Lebewesen aufweisen.

Gegenstand der Erfindung sind daher Amide der Formel (I) und deren Salze, 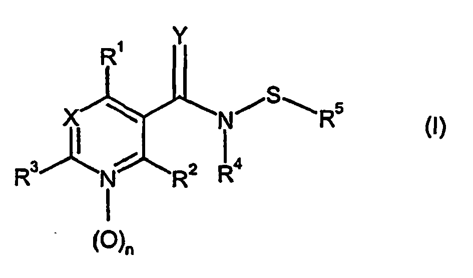 wobei die Symbole und Indizes folgende Bedeutungen haben:
- X: ist =CH- oder =N-;
- Y: ist =O oder =S;
- n: ist 0 oder 1;
- R¹: ist (C₁-C₆)-Alkyl, (C₁-C₆)-Haloalkyl, -S(Halogen)₅ oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
- R², R³: sind unabhängig voneinander Wasserstoff, (C₁-C₆)-Alkyl, (C₁-C₆)-Haloalkyl oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
- R⁴: ist Wasserstoff, (C₁-C₁₀)-Alkyl. (C₃-C₁₀)-Cycloalkyl, (C₃-C₁₀)-Alkenyl, (C₃ C₁₀)-Alkinyl, (C₆-C₁₄)-Aryl, (C₃-C₁₀)-Heterocyclyl oder (C₁-C₁₀)-Alkanoyl, wobei die genannten Reste gegebenenfalls ein- oder mehrfach substituiert sein können;
- R⁵: ist Wasserstoff, (C₁-C₁₀)-Alkyl, (C₃-C₁₀)-Alkenyl, (C₃-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl, wobei die genannten Reste gegebenenfalls ein- oder mehrfach substituiert sein können;
wobei Verbindungen der Formel (1), worin X =CH-, und R⁵ gegebenenfalls substituiertes (C₁-C₁₀)-Alkyl bedeuten, ausgeschlossen sind.

Weiterer Gegenstand der Erfindung sind Iminderivate der Formel (II) und deren salze, 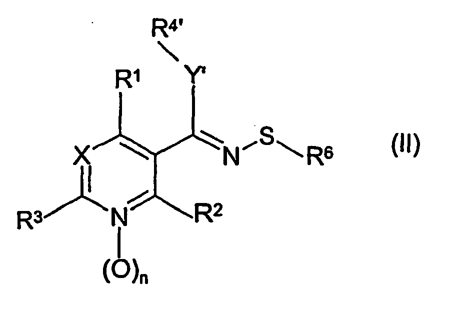 wobei die Symbole und Indizes folgende Bedeutungen haben:
- X: ist =CH- oder =N-, -O-, -S-;
- Y': ist -O- oder -S-;
- n: ist 0 oder 1;
- R¹: ist (C₁-C₆)-Alkyl, (C₁-C₆)-Haloalkyl, -S(Halogen)₅ oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
- R², R³: sind unabhängig voneinander Wasserstoff, (C₁-C₆)-Alkyl, (C₁-C₆)-Haloalkyl oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
- R^{4'}: ist Wasserstoff, (C₁-C₁₀)-Alkyl, (C₃-C₁₀)-Cycloalkyl, (C₃-C₁₀)-Alkenyl, (C₃-C₁₀)-Alkinyl, (C₆-C₁₄)-Aryl oder (C₃-C₁₀)-Heterocyclyl, wobei die genannten Reste gegebenenfalls ein- oder mehrfach substituiert sein können;
- R⁶: ist Wasserstoff, (C₁-C₁₀)-Alkyl, (C₂-C₁₀)-Alkenyl, (C₂-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl, wobei die genannten Reste gegebenenfalls ein- oder mehrfach substituiert sein können.

Bevorzugt haben die Symbole und Indizes in Formel (I) folgende Bedeutungen:
- X: ist vorzugsweise =CH-;
- Y: ist vorzugsweise =O.
- n: ist vorzugsweise 0.
- R¹: ist vorzugsweise SF₅, (C₁-C₆)-Haloalkyl, insbesondere ein- oder mehrfach durch F und/oder Cl substituiertes (C₁-C₆)-Alkyl, besonders bevorzugt SF₅, CF₃, CHF₂ oder CF₂Cl, ganz besonders bevorzugt CF₃.
- R², R³: sind vorzugsweise Wasserstoff, Halogen, (C₁-C₆)-Alkyl, (C₁-C₆)-Alkoxy, NH(C₁-C₆)-Alkyl, N(C₁-C₆)₂-Alkyl, besonders bevorzugt Wasserstoff;
- R⁴: ist vorzugsweise Wasserstoff, (C₁-C₆)-Alkyl oder ein- oder mehrfach durch F und/oder Cl substituiertes (C₁-C₆)-Alkyl, besonders bevorzugt Wasserstoff oder CH₃.
- R⁵: ist vorzugsweise (C₁-C₆)-Alkyl, (C₃-C₆)-Alkenyl, (C₃-C₆)-Alkinyl, (C₃-C₈)-Cycloalkyl, (C₆-C₁₄)-Aryl oder Heterocyclyl mit insgesamt ein bis drei Stickstoff-, Sauerstoff- und/oder Schwefelringatomen, wobei die genannten Reste gegebenenfalls ein- oder mehrfach substituiert sein können.

Besonders bevorzugt sind solche Verbindungen der Formel (I), für die die Symbole und Indizes folgende Bedeutung haben:
- X: ist vorzugsweise =CH-;
- Y: ist vorzugsweise =O;
- n: ist vorzugsweise 0;
- R¹: ist vorzugsweise -CF₃;
- R² und R³: sind vorzugsweise Wasserstoff;
- R⁵: ist vorzugsweise (C₁-C₁₀)-Alkyl. (C₂-C₁₀)-Alkenyl, (C₂-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl, wobei die genannten Reste gegebenenfalls ein- oder mehrfach substituiert sein können.

Bevorzugt haben die Symbole und Indizes in Formel (II) folgende Bedeutungen:
- X: ist vorzugsweise =CH-.
- Y': ist vorzugsweise -O-.
- n: ist vorzugsweise 0.
- R¹: ist vorzugsweise SF₅, (C₁-C₆)-Haloalkyl, insbesondere ein- oder mehrfach durch F und/oder Cl substituiertes (C₁-C₆)-Alkyl, besonders bevorzugt SF₅, CF₃, CHF₂ oder CF₂Cl, ganz besonders bevorzugt CF₃.
- R² R³: sind vorzugsweise Wasserstoff, Halogen, (C₁-C₆)-Alkyl, (C₁-C₆)-Alkoxy, NH(C₁-C₆)-Alkyl, N(C₁-C₆)₂-Alkyl, besonders bevorzugt Wasserstoff;
- R^{4'}: ist vorzugsweise (C₁-C₆)-Alkyl oder ein- oder mehrfach durch F und/oder Cl substituiertes (C₁-C₁)-Alkyl, besonders bevorzugt (C₁-C₆)-Alkyl.
- R⁶: ist vorzugsweise (C₁-C₁₀)-Alkyl, (C₃-C₈)-Cycloalkyl, Aryl, Benzyl oder Heterocyclyl, wobei die genannten Reste gegebenenfalls ein- oder mehrfach substituiert sein können.

Als Substituenten an den Resten R⁴, R^{4'}, R⁵ und R⁶ sind bevorzugt Gruppen R⁷, mit der folgenden Bedeutung:
- R⁷: ist gleich oder verschieden R⁸ oder zwei Reste R⁷ bilden zusammen mit den Atomen, an die sie gebunden sind, ein drei- bis achtgliedriges, gesättigtes oder ungesättigtes, gegebenenfalls mit einem oder mehreren Resten R⁸ substituiertes Ringsystem, das gegebenenfalls auch weitere Heteroatome, vorzugsweise O, N, S, SO und/oder SO₂, enthält;
- R⁸: ist gleich oder verschieden R⁹, R¹⁰, -C(W)R⁹, -C(=NOR⁹)R⁹, -C(=NNR⁹₂)R⁹, -C(=W)OR⁹, -C(=W)NR⁹₂, -OC(=W)R⁹, -OC(=W)OR⁹, -NR⁹C(=W)R⁹, -N[C(=W)R⁹]₂, -NR⁹C(=W)OR⁹, -C(=W)NR⁹-NR⁹₂, -C(=W)NR⁹-NR⁹[C(=W)R⁹], -NR⁹-C(=W)NR⁹_{2'} -NR⁹-NR⁹C(=W)R⁹, -NR⁹-N[C(=W)R⁹]₂, -N[(C=W)R⁹]-NR⁹₂, -NR⁹-N[(C=W)WR⁹], -NR⁹[(C=W)NR⁹₂], -NR⁹(C=NR⁹)R⁹, -NR⁹(C=NR⁹)NR⁹_{2'} -O-NR⁹₂, -O-NR⁹(C=W)R⁹, -SO₂NR⁹₂, -NR⁹SO₂R⁹, -SO₂OR⁹, -OSO₂R⁹, -OR⁹, -NR⁹₂, -SR⁹, -SiR⁹₃, -PR⁹₂, -P(=W)R⁹₂, -SOR⁹, -SO₂R⁹, -PW₂R⁹₂, -PW₃R⁹₂ oder zwei Reste R⁸ sind zusammen (=W), (=N-R⁹), (= CR₂⁹), (= CHR⁹), oder (=CH₂);
- W: ist = o oder = s;
- R⁹: ist gleich oder verschieden (C₁-C₆)-Alkyl, (C₂-C₆)-Alkenyl, (C₂-C₆)-Alkinyl, (C₃-C₈)-Cycloalkyl, (C₄-C₈)-Cycloalkenyl, (C₃-C₈)-Cycloalkyl-(C₁-C₄)-Alkyl, (C₄-C₈)-Cycloalkenyl-(C₁-C₄)-Alkyl, (C₃-C₈)-Cycloalkyl-(C₂-C₄)-Alkenyl, (C₄-C₈)-Cycloalkenyl-(C₂-C₄)-Alkenyl, (C₁-C₆)-Alkyl-(C₃-C₈)-Cycloalkyl, (C₂-C₆)-Alkenyl-(C₃-C₈)-Cycloalkyl, (C₂-C₆)-Alkinyl-(C₃-C₈)-Cycloalkyl. (C₁-C₆)-Alkyl-(C₄-C₈)-Cycloalkenyl, (C₂-C₆)-Alkenyl-(C₄-C₈)-Cycloalkenyl, Aryl, Heterocyclyl; wobei die genannten Reste gegebenenfalls mit einem oder mehreren Resten R¹⁰ substituiert sind und gegebenenfalls zwei Reste R⁹ zusammen ein Ringsystem bilden;
- R¹⁰: ist gleich oder verschieden Halogen, Cyano, Nitro, Hydroxy, Thio, Amino, Formyl, (C₁-C₆)-Alkanoyl, (C₁-C₆)-Alkoxy, (C₃-C₆)-Alkenyloxy, (C₃-C₆)-Alkinyloxy, (C₁-C₆)-Haloalkyloxy, (C₃-C₆) Haloalkenyloxy, (C₃-C₆)-Haloalkinyloxy, (C₃-C₈)-Cycloalkoxy, (C₄-C₈)-Cycloalkenyloxy, (C₃-C₈)-Halocycloalkoxy, (C₄-C₈)-Halocycloalkenyloxy, (C₃-C₈)-Cycloalkyl-(C₁-C₄)-Alkoxy, (C₄-C₈)-Cycloalkenyl-(C₁-C₄)-Alkoxy, (C₃-C₈)-Cycloalkyl-(C₂-C₄)-Alkenyloxy, (C₄-C₈)-Cycloalkenyl-(C₁-C₄)-Alkenyloxy, (C₁-C₆)-Alkyl-(C₃-C₈)-Cycloalkoxy, (C₂-C₆)-Alkenyl-(C₃-C₈)-Cycloalkoxy, (C₂-C₆)-Alklnyl-(C₃-C₈)-Cycloalkoxy, (C₁-C₆)-Alkyl-(C₄-C₈)-Cycloalkenyloxy, (C₂-C₆)-Alkenyl-(C₄-C₈)-Cycloalkenyloxy, (C₁-C₄)-Alkoxy-(C₁-C₆)-Alkoxy, (C₁-C₄)-Alkoxy-(C₃-C₆)-Alkenyloxy, Carbamoyl, (C₁-C₆)-Mono- oder -Dialkylcarbamoyl, (C₁-C₆)-Mono-oder -Dihaloalkylcarbamoyl, (C₃-C₈)-Mono- oder -Dicycloalkylcarbamoyl, (C₁-C₆)-Alkoxycarbonyl, (C₃-C₈)-Cycloalkoxycarbonyl, (C₁-C₆)-Alkanoyloxy, (C₃-C₈)-Cycloalkanoyloxy, (C₁-C₆)-Haloalkoxycarbonyl, (C₁-C₆)-Haloalkanoyloxy, (C₁-C₆)-Alkanoylamino, (C₁-C₆)-Haloalkanoylamino, (C₂-C₆)-Alkenoylamino, (C₃-C₈)-Cycloalkanoylamino, (C₃-C₈)-Cycloalkyl-(C₁-C₄)-Alkanoylamino, (C₁-C₆)-Alkylthio, (C₃-C₆)-Alkenylthio, (C₃-C₆)-Alkinylthio, (C₁-C₆)-Haloalkylthio, (C₃-C₆)-Haloalkenylthio, (C₃-C₆)-Haloalkinylthio, (C₃-C₈)-Cycloalkylthio, (C₄-C₈)-Cycloalkenylthio, (C₃-C₈)-Halocycloalkthlo, (C₄-C₈)-Halocycloalkenylthio, (C₃-C₈)Cycloalkyl-(C₁-C₄)-Alkylthio, (C₄-C₈)-Cycloalkyl-( C₁-C₄)-Alkylthio, (C₃-C₈)-Cycloalkyl-(C₃-C₄)-Alkenylthio, (C₄-C₈)-Cycloalkenyl-(C₃-C₄)-Alkenylthio, (C₁-C₆)-Alkyl-(C₃-Cₛ)-Cycloalkylthio, (C₂-C₆)-Alkenyl-(C₃-C₈)-Cycloalkylthio. (C₂-C₆)-Alkinyl-(C₃-C₈) Cycloalkylthio, (C₁-C₆)-Alkyl-(C₄-C₈)-Cycloalkenylthio, (C₂-C₆)-Alkenyl-(C₄-C₈)-Cycloalkenylthio, (C₁-C₆)-Alkylsulfinyl, (C₃-C₆)-Alkenylsulfinyl, (C₃-C₈)-Alkinylsulfinyl, (C₁-C₆)-Haloalkylsulfinyl, (C₃-C₆)-Haloalkenylsulfinyl, (C₃-C₆)-Haloalkinylsulflnyl, (C₃-C₈)-Cycloalkylsulfinyl, (C₄-C₈)-Cycloa,lkenylsulfinyl, (C₃ C₈)-Halocycloalkylsulfinyl, (C₄-C₈)-Halocycloalkenylsulfinyl, (C₃-C₈)-Cycloalkyl-(C₁-C₄)-Alkylsulfinyl, (C₄-C₈)-Cycloalkenyl-(C₁-C₄)-Alkylsulfinyl, (C₃-C₈)-Cycloalkyl-(C₃-C₄)-Alkenylsulfinyl, (C₄-C₈)-Cycloalkenyl-(C₃-C₄)-Alkenylsulfinyl, (C₁-C₆)-Alkyl-(C₃-C₈)-Cycloalkylsulfinyl, (C₂-C₆)-Alkenyl-(C₃-C₈)-Cycloalkylsulfinyl, (C₂-C₆)-Alkinyl-(C₃-C₈)-Cycloalkylsulfinyl, (C₁-C₆)-Alkyl-(C₄-C₈)-Cycloalkenylsulfinyl, (C₂-C₆)-Alkenyl-(C₄-C₈)-Cycloalkenylsulfinyl, (C₁-C₆)-Alkylsulfonyl, (C₃-C₆)-Alkenylsulfonyl, (C₃-C₆)-Alkinylsulfonyl, (C₁-C₆)-Haloalkylsulfonyl, (C₃-C₆)-Haloalkenylsulfonyl, (C₃-C₆)Haloalkinylsulfonyl, (C₃-C₈)-Cycloalkylsulfonyl, (C₄-C₈)-Cycloalkenylsulfonyl, (C₃-C₈)-Halocycloalkylsulfonyl, (C₄-C₈)-Halocycloalkenylsulfonyl, (C₃-C₈)-Cycloalkyl-(C₁-C₄)-Alkylsulfonyl, (C₄-C₈)-Cycloalkenyl-(C₁-C₄)-Alkylsulfonyl, (C₃-C₈)-Cycloalkyl-(C₃-C₄)-Alkenylsulfonyl, (C₄-C₈)-Cycloalkenyl-(C₃-C₄)-Alkenylsulfonyl, (C₁-C₆)-Alkyl-(C₃-C₈)-Cycloalkylsulfonyl, (C₂₋C₆)-Alkenyl-(C₃-C₈)-Cycloalkylsulfonyl, (C₂-C₆)-Alkinyl-(C₃-C₈)-Cycloalkylsulfonyl, (C₁-C₆)-Alkyl-(C₄-C₈)-Cycloalkenylsulfonyl, (C₂-C₆)-Alkenyl-(C₄-C₈)-Cycloalkenylsulfonyl, (C₁-C₆)-Dialkylamino, (C₁-C₆)-Alkylamino, (C₃-C₆)-Alkenylamino. (C₃ C₆)-Alkinylamino, (C₁-C₆)-Haloalkylamino, (C₃-C₆)-Haloalkenylamino, (C₃-C₆)-Haloalkinylamino, (C₃-C₈)-Cycloalkylamino, (C₄-C₈)-Cycloalkenylamino, (C₃-C₈)-Halocycloalkamino, (C₄ C₈)-Halocycloalkenylamino, (C₃-C₈)-Cycloalkyl-(C₁-C₄)-Alkylamino, (C₄-C₈)-Cycloalkenyl-(C₁-C₄)-Alkylamino, (C₃-C₈)-Cycloalkyl-(C₃-C₄)-Alkenylamino, (C₄-C₈)-Cycloalkenyl-(C₃-C₄)-Alkenylamino, (C₁-C₆)-Alkyl-(C₃-C₈)-Cycloalkylamino, (C₂-C₆)-Alkenyl-(C₃-C₈)-Cycloalkylamino, (C₂-C₆)-Alkinyl-(C₃-C₆)-Cycloalkylamino, (C₁-C₆)-Alkyl-(C₄-C₈)-Cycloalkenylamino, (C₂-C₆)-Alkenyl-(C₄-C₈)-Cycloalkenylamino, (C₁-C₆)-Trialkylsilyl, Aryl, Aryloxy, Arylthio, Arylsulfinyl, Arylsulfonyl, Arylamino, Aryl-(C₁-C₄)-Alkoxy, Aryl-(C₃-C₄)-Alkenyloxy, Aryl-(C₁-C₄)-Alkylthio, Aryl-(C₁-C₄)-Alkylsulfinyl, Aryl-(C₁-C₄)-Alkylsulfonyl, Aryl-(C₂-C₄)-Alkenylthio, Aryl-(C₂-C ₄)-Alkenylsulfinyl, Aryl-(C₂-C₄)-Alkenylsulfonyl, Aryl-(C₁-C₄)-Alkylamino, Aryl-(C₃-C₄)-Alkenylamino, Aryl-(C₁-C₆)-Dialkylsilyl, Diaryl-(C₁-C₆)-Alkylsilyl, Triarylsilyl und 5- oder 6-gliedriges Heterocyclyl, wobei der cyclische Teil der vierzehn letztgenannten Reste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino und (C₁-C₄)-Alkanoyl substituiert ist; und, falls R⁹ Aryl oder Heterocyclyl ist, (C₁-C₄)-Alkyl oder (C₁-C₄)-Haloalkyl.
- R¹⁰: ist bevorzugt gleich oder verschieden Halogen, Cyano, Nitro, (C₁-C₆)-Alkanoyl, (C₁-C₆)-Alkoxy, (C₁-C₆)-Haloalkyloxy, (C₃-C₈)-Cycloalkoxy, (C₃-C₈)-Cycloalkyl-(C₁-C₄)-Alkoxy, (C₁-C₆)-Mono- oder -Dialkylcarbamoyl, (C₁-C₆)-Alkoxycarbonyl, (C₁-C₆)-Haloalkoxycarbonyl, (C₁-C₆)-Alkylthio, (C₁-C₆)-Haloalkylthio, (C₃-C₈)-Cycloalkylthio, (C₁-C₆)-Alkylsulfinyl, (C₁-C₆)-Haloalkylsulfinyl, (C₃-C₈)-Cycloalkylsulfinyl, (C₁-C₆)-Alkylsulfonyl, (C₁-C₆)-Haloalkylsulfonyl, (C₃-C₈)-Cycloalkylsulfonyl, (C₁-C₆)-Dialkylamino, (C₁-C₆)-Alkylamino, (C₃-C₈)-Cycloalkylamino, (C₁-C₆)-Trialkylsilyl, Aryl, Aryloxy, Arylthio, Aryl-(C₁-C₄)-alkyl, Arylamino, Aryl-(C₁-C₄)-Alkoxy, wobei der cyclische Teil der sechs letztgenannten Reste gegebenenfalls durch einen oder mehrere Reste aus der Gruppe Halogen, Nitro, (C₁-C₄)-Alkyl, (C₁-C₄)-Haloalkyl, (C₁-C₄)-Alkoxy und (C₁-C₄)-Haloalkoxy substituiert ist; und, falls R⁹ Aryl oder Heterocyclyl ist, (C₁-C₄)-Alkyl oder (C₁-C₄)-Haloalkyl.

Besonders bevorzugt unter den Resten R⁵ sind Phenylreste, die gegebenenfalls ein-oder mehrfach substituiert sind; insbesondere solche der Formel (IIa) 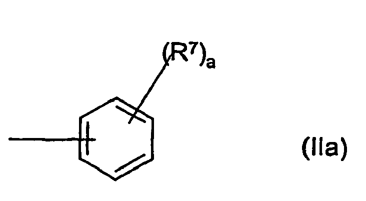 wobei die Symbole und Indizes folgende Bedeutungen haben:
- R⁷: hat die oben angegebenen Bedeutungen;
- a: ist 0, 1, 2, 3 oder 4, vorzugsweise 0, 1 oder 2.

Die Bezeichnung "Halogen" umfaßt Fluor, Chlor, Brom und Iod. Vorzugsweise handelt es sich um Chlor oder um Fluor.

Die Bezeichnung "S(Halogen)₅" umfasst die Gruppen -SI₅, -SBr₅, -SCl₅ und insbesondere -SF₅.

Unter dem Ausdruck "(C₁-C₆)-Alkyl" ist ein unverzweigter oder verzweigter Kohlenwasserstoffrest mit ein bis sechs Kohlenstoffatomen, wie z.B. der Methyl-, Ethyl-, Propyl-, Isopropyl-, 1-Butyl-, 2-Butyl-, 2-Methylpropyl-, tert.-Butyl-, 1-Pentyl, 2-Methylbutyl-, 1,1-Dimethylpropyl- oder 1-Hexylrest zu verstehen. Entsprechend ist unter Alkylresten mit einem größeren Bereich an Kohlenstoffatomen ein unver-zweigter oder verzweigter gesättigter Kohlenwasserstoffrest zu verstehen, der eine Anzahl an Kohlenstoffatomen enthält, die dieser Bereichsangabe entspricht. Der Ausdruck "(C₁-C₁₀)-Alkyl" umfaßt demnach die vorgenannten Alkylreste, sowie z.B. den Heptyl-, Octyl-, 2-Ethylhexyl-, Nonyl- oder Decylrest.

Unter "(C₁-C₆)-Haloalkyl" ist eine unter dem Ausdruck "(C₁-C₆)-Alkyl" genannte Alkylgruppe zu verstehen, in der ein oder mehrere Wasserstoffatome durch die gleiche Anzahl gleicher oder verschiedener Halogenatome, bevorzugt Chlor oder Fluor, ersetzt sind, wie die Trifluormethyl-, die 1-Fluorethyl-, die 2,2,2-Trifluorethyl-, die Chlormethyl-, Fluormethyl-, die Difluormethyl- und die 1,1,2,2-Tetrafluorethylgruppe.

Unter "(C₁-C₆)-Alkoxy" ist eine Alkoxygruppe zu verstehen, deren Kohlenwasserstoffrest die unter dem Ausdruck "(C₁-C₆)-Alkyl" angegebene Bedeutung hat. Sinngemäß sind Alkoxygruppen zu verstehen, die einen größeren Bereich an Kohlenstoffatomen umfassen.

Die Bezeichnungen "Alkenyl" und "Alkinyl" mit einer vorangestellten Bereichsangabe von Kohlenstoffatomen bedeuten einen geradkettigen oder verzweigten Kohlenwasserstoffrest mit einer dieser Bereichsangabe entsprechenden Kohlenstoffatomzahl, der mindestens eine Mehrtachbindung beinhaltet, wobei sich diese an beliebiger Position des betreffenden ungesättigten Restes befinden kann. "(C₂-C₄)-Alkenyl" steht demnach z.B. für die Vinyl-, Allyl-, 2-Methyl-2-propenyl- oder 2-Butenyl-Gruppe; "(C₂-C₆)-Alkenyl" steht für die vorstehend genannten Reste sowie z.B. für die Pentenyl-, 2-Methylpentenyl- oder die Hexenyl-Gruppe. "(C₂-C₄)-Alkinyl" steht z.B. für die Ethinyl-, Propargyl-, 2-Methyl-2-propinyl- oder 2-Butinyl-Gruppe. Unter "(C₂-C₆)-Alkinyl" sind die vorstehend genannten Reste sowie z.B. die 2-Pentinyl- oder die 2-Hexinyl-Gruppe und unter "(C₂-C₁₀)-Alkinyl" die vorstehend genannten Reste sowie z.B. die 2-Octinyl- oder die 2-Decinyl-Gruppe zu verstehen.

"(C₃-C₈)-Cycloalkyl" steht für monocyclische Alkylreste, wie den Cyclopropyl-, Cyclobutyl-, Cyclopentyl-, Cyclohexyl-, Cycloheptyl- oder Cyclooctylrest und für bicyclische Alkylreste, wie den Norbomylrest.

Unter dem Ausdruck "(C₃-C₈)-Cycloalkyl-(C₁-C₄)-alkyl " ist beispielsweise der Cyclopropylmethyl-, Cyclopentylmethyl-, Cyclohexylmethyl-, Cyclohexylethyl- und Cyclohexylbutyl-Rest und unter dem Ausdruck "(C₁-C₆)-Alkyl-(C₃-C₈)-cycloalkyl" beispielsweise der 1-Methyl-cyclopropyl-,1-Methyl-cyclopentyl-, 1-Methylcyclohexyl, 3-Hexyl-cyclobutyl- und 4-tert.-Butyl-cyclohexyl-Rest zu verstehen.

"(C₁-C₄)-Alkoxy-(C₁-C₆)-alkyloxy" bedeutet eine wie vorstehend definierte Alkoxy-Gruppe, die durch eine weitere Alkoxy-Gruppe substituiert ist, wie z.B. 1-Ethoxyethoxy.

Unter "(C₃-C₈)-Cycloalkoxy" oder "(C₃-C₈)-Cycloalkylthio" ist einer der oben angeführten (C₃-C₈) Cycloalkyl-Reste, der über ein Sauerstoff- oder Schwefelatom verknüpft ist, zu verstehen.

"(C₃-C₈)-Cycloalkyl-(C₁-C₆)-alkoxy" bedeutet z.B. die Cyclopropylmethoxy, Cyclobutylmethoxy-, Cyclopentylmethoxy-, Cyclohexylmethoxy-, Cyclohexylethoxy-oder die Cyclohexylbutoxy-Gruppe.

Der Ausdruck "(C₁-C₄)-Alkyl-(C₃ C₈)-cycloalkoxy" steht z.B. für die Methylcyclopropyloxy-, Methylcyclobutyloxy- oder die Butylcyclohexyloxy-Gruppe.

"(C₁-C₆)-Alkylthio" steht für eine Alkylthiogruppe, deren Kohlenwasserstoffrest die unter dem Ausdruck "(C₁-C₆)-Alkyl" angegebene Bedeutung hat.

Analog bedeuten "(C₁-C₆)-Alkylsulfinyl" z.B. die Methyl-, Ethyl-, Propyl-, Isopropyl-, Butyl-, Isobutyl-, sek.-Butyl- oder tert.-Butylsulfinyl-Gruppe und "(C₁-C₆)-Alkylsulfonyl" z.B. die Methyl-, Ethyl-, Propyl-, Isopropyl-, Butyl-, Isobutyl-, sek.-Butyl- oder tert.-Butylsulfonyl-Gruppe.

"(C₁-C₆)-Alkylamino" steht für ein Stickstoffatom, das durch ein oder zwei, gleiche oder verschiedene Alkylreste der obigen Definition substituiert ist.

Der Ausdruck "(C₁-C₆)-Mono- oder -Dialkylcarbamoyl" bedeutet eine Carbamoylgruppe mit einem oder zwei Kohlenwasserstoffresten, die die unter dem Ausdruck "(C₁-C₈)-Alkyl" angegebene Bedeutung haben und die im Fall von zwei Kohlenwasserstoffresten gleich oder verschieden sein können.

Analog bedeutet "(C₁-C₆)-Dihaloalkylcarbamoyl" eine Carbamoylgruppe, die zwei (C₁-C₆)-Haloalkylreste gemäß der obigen Definition oder einen (C₁-Cₑ)-Haloalkylrest und einen (C₁-C₆)-Alkylrest gemäß der obigen Definition trägt.

"(C₁-C₆)-Alkanoyl" steht z.B. für die Formyl-, Acetyl-, Propionyl-, Butyryl- oder 2-Methylbutyryl-Gruppe.

Unter dem Ausdruck "Aryl" ist ein carbocyclischer, d.h. aus Kohlenstoffatomen aufgebauter, aromatischer Rest mit vorzugsweise 6 bis 14, insbesondere 6 bis 12 C-Atomen, wie beispielsweise Phenyl, Naphthyl oder Biphenylyl, vorzugsweise Phenyl zu verstehen.

"Aroyl" bedeutet demnach einen wie vorstehend definierter Arylrest, der über eine Carbonyl-Gruppe gebunden ist, wie z.B. die Benzoyl-Gruppe.

Der Ausdruck "Heterocyclyl" steht vorzugsweise für einen cyclischen Rest, der vollständig gesättigt, teilweise ungesättigt oder vollständig ungesättigt bzw. aromatisch sein kann und der durch mindestens ein oder mehrere gleiche oder verschiedene Atome aus der Gruppe Stickstoff, Schwefel oder Sauerstoff unterbrochen sein kann, wobei jedoch nicht zwei Sauerstoffatome direkt benachbart sein dürfen und noch mindestens ein Kohlenstoffatom im Ring vorhanden sein muß, wie z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, 4H-Chinolizin, Piperidin, Pyrrolidin, Oxazolin, Tetrahydrofuran, Tetrahydropyran, Isoxazolidin oder Thiazolidin. Der Ausdruck "Heteroaromat" umfaßt demnach von den vorstehend unter "Heterocyclyl" genannten Bedeutungen jeweils die vollständig ungesättigten aromatischen heterocyclischen Verbindungen.

Heterocyclyl bedeutet besonders bevorzugt ein gesättigtes, teilgesättigtes oder aromatisches Ringsystem mit 3 bis 6 Ringgliedern und 1 bis 4 Heteroatomen aus der Gruppe O, S und N, wobei mindestens ein Kohlenstoffatom im Ring vorhanden sein muß.

Ganz besonders bevorzugt bedeutet Heterocyclyl ein Radikal des Pyridin, Pyrimidin, (1,2,4)-Oxadiazol, (1,3,4)-Oxadiazol, Pyrrol, Furan, Thiophen, Oxazol, Thiazol, Imidazol, Pyrazol, Isoxazol, 1,2,4-Triazol, Tetrazol, Pyrazin, Pyridazin, Oxazolin, Thiazolin, Tetrahydrofuran, Tetrahydropyran, Morpholin, Piperidin, Piperazin, Pyrrolin, Pyrrolidin, Oxazolidin, Thiazolidin, Oxiran und Oxetan.

"Aryl-(C₁-C₄)-alkoxy" steht für einen Ober eine (C₁-C₄)-Alkoxygruppe verknüpften-Arylrest, z.B. den Benzyloxy-, Phenylethoxy-, Phenylbutoxy- oder Naphthylmethoxy-Rest.

"Arylthio" bedeutet einen über ein Schwefelatom verknüpften Arylrest, z.B. den Phenylthio- oder den 1- oder 2-Naphthylthio-Rest. Analog bedeutet "Aryloxy" z.B. den Phenoxy- oder 1- oder 2-Naphthyloxy-Rest.

"Aryl-(C₁-C₄)-alkylthio" steht für einen Arylrest, der über einen Alkylthiorest verknüpft ist, z.B. den Benzylthio-, Naphthylmethylthio- oder den Phenylethylthio-Rest.

Der Ausdruck "(C₁-C₆)-Trialkylsilyl" bedeutet ein Siliciumatom, das drei gleiche oder verschiedene Alkylreste gemäß der obigen Definition trägt. Analog stehen "Aryl-(C₁-C₆)-Dialkylsilyl" für ein Siliciumatom, das einen Arylrest und zwei gleiche oder verschiedene Alkylreste gemäß der obigen Definition trägt, "Diaryl-(C₁-C₈)-Alkylsilyl" für ein Siliciumatom, das einen Alkylrest und zwei gleiche oder verschiedene Arylreste gemäß der obigen Definition trägt, und "Triarylsilyl" für ein Siliciumatom, das drei gleiche oder verschiedene Arylreste gemäß der obigen Definition trägt.

Zu den Substituenten, mit denen die verschiedenen aliphatischen, aromatischen und heterocyclischen Ringsysteme versehen sein können, zählen bevorzugt Halogen, Nitro, Cyano, Di-(C₁-C₄)-alkylamino, (C₁-C₄)-Alkyl, (C₁-C₄)-Trialkylsilyl, (C₁-C₄)-Alkoxy, (C₁-C₄)-Alkoxy-(C₁-C₄)-alkyl, (C₁-C₂)-Alkoxy-[CH₂CH₂]_{1,2}-ethoxy, (C₁-C₄)-Alkylthio, (C₁-C₄)-Alkylsulfinyl, (C₁-C₄)-Alkylsulfonyl, Phenyl, Benzyl, Phenoxy, Phenylthio, Halogenphenoxy, (C₁-C₄)-Alkylthiophenoxy, (C₁-C₄)-Alkoxyphenoxy, Phenylthio, Heterocyclyl, Heterocylylthio, Heterocyclyloxy, Halogenheterocyclyloxy, Alkylheterocyclyloxy oder Alkoxyheterocyclyloxy, wobei in den Alkylresten und den davon abgeleiteten Resten eines oder mehrere Wasserstoffatome, im Falle von Fluor auch bis zur Maximalanzahl durch Halogen, bevorzugt Chlor oder Fluor, ersetzt sein können.

Besonders bevorzugte Substituenten sind, insbesondere bei cyclischen Systemen, Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Atkylthio, (C₁-C₄)-Haloalkylthio; (C₁-C₄)-Alkylamino, (C₁-C₄)-Haloalkylamino und (C₁-C₄)-Alkanoyl.

Je nach Art der oben definierten Substituenten weisen die Verbindungen der allgemeinen Formel (I) und (II) saure oder basische Eigenschaften auf und können Salze bilden. Tragen die Verbindungen der allgemeinen Formel (I) und (II) beispielsweise Gruppen wie Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der allgemeinen Formel (I) und (II) beispielsweise Gruppen wie Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide, akarizide und mitizide Eigenschaften auf.

Die Verbindungen der allgemeinen Formel (I) und (II) können ein asymmetrisches Schwefelatom und/oder eines oder mehrere asymmetrische Kohlenstoffatome oder Stereoisomere an Doppelbindungen aufweisen. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Isomeren aufgetrennt werden. Racemate können nach üblichen Methoden in die Enantiomeren aufgetrennt werden.

Die Herstellung der erfindungsgemäßen Verbindungen erfolgt nach an sich literaturbekannten Methoden, wie sie in Standardwerken zur Organischen Synthese beschrieben werden (vgl. z. B. T. L. Gilchrist, C.J. Moody, Chem. Rev. 77, 409 (1977); Houben-Weyl, Methoden der Organischen Chemie, Bd. E11, S.877).

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) und (II) umsetzt.

Die vorliegende Erfindung betrifft auch Verfahren zur Herstellung von Verbindungen der allgemeinen Formel (I) und (II).

Verbindungen der allgemeinen Formel (I) lassen sich aus der Reaktion von Thiolen mit Nitrenbildenden Verbindungen, wie N-Chloramiden oder Aziden, erhalten. Dabei kann die Nitrenbildende Verbindung auch in der Lösung synthetisiert werden. Die Reaktion kann aber auch invers mit Halomerkaptan und Amid durchgeführt werden. Typische Lösungsmittel sind unter Reaktionsbedingungen inerte organische Lösungsmittel, die nicht protisch sein können, wie Toluol oder Acetonitril. Typischerweise werden Basen zur Reaktionslösung dazugegeben, das Salz kann aber auch vorgebildet eingesetzt werden.

Zur Herstellung von Verbindungen der allgemeinen Formel (I), in weichen R¹, R², R³, R⁴, R⁵, Y, n und X die zur Formel (I) angegebenen Bedeutungen haben wird beispielsweise so verfahren, daß man ein Carbonsäureamid der Formel (III), worin R¹, R², R³, R⁴, Y, n und X die in Formel (I) angegebenen-Bedeutungen haben, mit einem Halogenierungsmittel, bevorzugt einem Chlorierungs- oder Bromierungsmittel, zu einer Verbindung der Formel (IV) umsetzt, worin R¹, R², R³, R⁴, Y, n und X die zur Formel (I) angegebenen Bedeutungen haben und Hal Halogen, bevorzugt Chlor oder Brom, bedeutet, und diese Verbindung anschließend mit einem Thioether R⁵SH, in dem R⁶ die zur Formel (I) angegebenen Bedeutungen hat, in Gegenwart einer Base zu den Endprodukten der Formel (I) umsetzt:

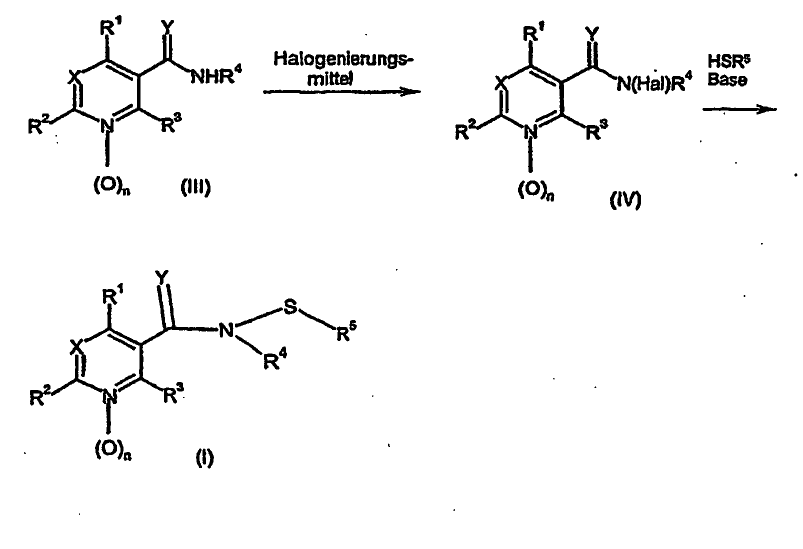

Geeignete Halogenierungsmittel zur Herstellung der Verbindungen (IV) sind beispielsweise Organo- oder Alkalihypochlorite, wie z.B. tert.-Butylhypochlorit oder Natrium- oder Kaliumhypochlorit, Alkalihypobromite wie Natrium- oder Kaliumhypobromit oder die elementaren Halogene in Gegenwart einer Base, wie z.B. Alkali- oder Erdalkalihydroxid oder-carbonat.

Die Herstellung von N-Chlor-4-trifluormethylnicotinamid und dessen Salzen der Formel (IVa) 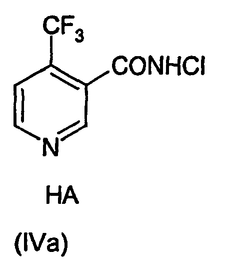 worin A ein nicht oxidierbares, organisches oder anorganisches Anion bedeutet, kann durch Chlorierung von 4-Trifluormethylnicotinsäureamid mit Cl₂ in wässeriger Säure und gegebenenfalls darauffolgenden Anionenaustausch und/oder gegebenenfalls Umsetzung mit einer Base zu N-Chlor-4-trifluormethylnicotinamid erfolgen.

"Nicht oxidierbar" bedeutet im Sinne dieser Beschreibung, daß das entsprechende Anion nicht mit der N-Cl-Gruppe des N-Chlor-4-trifluormethylnicotinamids reagiert.

A ist vorzugsweise F, HF₂, Cl, BF₄, PF₆, HSO₄, 1/2 SO₄, CH₃COO, CF₃COO, CF₃SO₃, CH₃SO₃, p-CH₃-C₆H₅SO₃ oder H₂PO₄.

Die Ausgangsverbindung, 4-Trifluormethylnicotinamid, bzw. deren N-substituierte Analoge sind bekannt und mit ihrer Herstellung beispielsweise in EP-A-580,374 und DE-A 100 619 67 beschrieben.

Die Reaktionstemperatur liegt üblicherweise zwischen -5 °C und +40 °C, bevorzugt zwischen 0 °C und +25 °C.

Das Verfahren wird in einer wässerigen Säure, beispielsweise HCl, H₂SO₄, HBF₄, CH₃C0OH oder CF₃COOH, vorzugsweise HCl (bevorzugte Konzentration 3-10 Gew.%) durchgeführt. Man kann auch Gemische von mehreren Säuren verwenden.

Man setzt Cl₂, vorzugsweise gasförmig, ein; im allgemeinen in Mengen von 1 bis 1,5 Mol, insbesondere 1 bis 1,3 Mol, bevorzugt 1 bis 1,2 Mol, bezogen auf 1 Mol 4-Trifluormethylnicotinsäureamid.

Die Chlorierung von 4-Trifluormethylnicotinsäureamid führt zu dem entsprechenden Salz, vorzugsweise dem Hydrochlorid.

Die Aufarbeitung erfolgt nach dem Fachmann geläufigen Methoden, beispielsweise wird das ausgefallene Produkt abfiltriert, gewaschen und getrocknet.

Ein nachfolgender Anionenaustausch kann nach bekannten dem Fachmann geläufigen Methoden erfolgen. Beispielsweise kann man das bei der Reaktion erhaltene Salz in einem geeigneten Lösungsmittel auflösen, in dem das später gewünschte Salz unlöslich ist. Durch Umsetzung mit einem in diesem Lösungmittel ebenfalls löslichen Salz, welches das gewünschte Anion enthält, erhält man durch Ausfällung das gewünschte Salz, da es im gewählten Lösungsmittel nicht löslich ist.

Falls gewünscht kann in einfacher, dem Fachmann geläufiger Weise durch Umsetzung mit Base die freie N-Chlorverbindung freigesetzt werden.

Geeignete Basen sind beispielsweise Hydroxide, Carbonate, Hydrogencarbonate, Acetate der Alkali- und Erdalkalimetalle, insbesondere die von Natrium, Kalium, Magnesium und Calcium, weiterhin tertiäre Amine mit (C₁-C₄)-Alkylresten. Ferner ist es möglich, die freie Base durch Behandlung mit Wasser und Extraktion mit organischem Lösemitteln zu isolieren.

Die Umsetzung der N-Halogenamide (IV), gegebenenfalls auch als Salz, zu den Endprodukten (1) erfolgt z.B. in einem inerten Lösemittel, wie z.B. Dichlormethan, Chloroform, Tetrachlormethan oder Benzol, in einem Temperaturbereich zwischen 0°C und 100°C, bevorzugt 20°C und 50°C und in Gegenwart einer Base.

Geeignete Basen sind z. B. Alkali- oder Erdaikalimetall-hydroxide, -carbonate oder -hydrogencarbonate oder organische Basen, wie z. B. Trialkylamine oder Pyridin.

Die beschriebene Reaktionssequenz kann gegebenenfalls auch als Eintopfreaktion durchgeführt werden, wobei auch Intermediate der Formel (V), in der R⁵ die oben zur Formel (I) angebenen Bedeutung hat und Z ein Halogenrest, bevorzugt Chlor oder Brom ist, als Reaktionpartner des Amids (III) auftreten können.

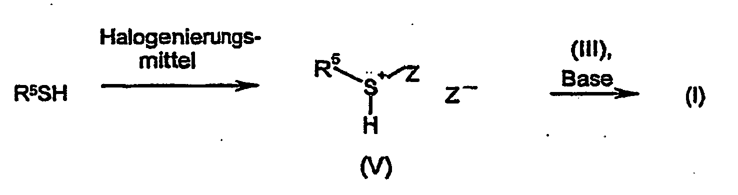

Zur Herstellung von Verbindungen der Formel (I), In welchen R¹, R², R³, R⁴, R⁵, Y, n und X die zur Formel (I) angegebenen Bedeutungen haben, kann weiterhin so verfahren werden, daß man eine Carbonsäure oder Thiocarbonsäure der Formel (VI).

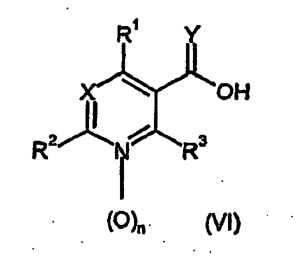

in der R¹, R², R³, Y, X und n die zur Formel (I) angegebenen Bedeutungen haben, in Form eines aktivierten Derivats dieser Säure, in Gegenwart einer Base, mit einer Verbindung der Formel (VII), worin R⁴, R⁵ die zur Formel (I) angegebenen Bedeutungen haben, 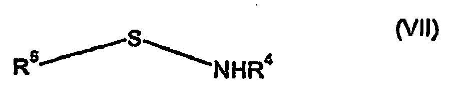 umsetzt.

Als aktiviertes Derivat der Säure kann beispielsweise ein Anhydrid, Azolid oder bevorzugt ein Säurechlorid eingesetzt werden. Als Basen eignen sich beispielsweise Amine, wie Triethylamin, Diisopropylethylamin. Pyridin oder Lutidin oder auch Alkali-oder Erdalkalimetallhydroxide, -carbonate oder -hydrogencarbonate. Die Reaktion wird vorteilhaft in einem inerten Lösemittel wie z. B. Dichlormethan, Chloroform, Tetrachlokohlenstoff, Benzol, Toluol, Diethylether oder Tetrahydrofuran oder auch in Gemischen dieser Lösemittel in-einem Temperaturbereich zwischen 0°C und.100°C, bevorzugt 20°C und **50°C** durchgeführt.

Verbindungen der Formel (la), **worin R⁴ Wasserstoff** bedeutet und R⁵ eine der oben definierten Gruppen darstellt, die ein β-Wasserstoffatom aufweist, vorzugsweise eine verzweigte Alkylgruppe mit einem ein β-Wasserstoftatom ist, lassen sich auch durch thermische Zersetzung der entsprechenden Sulfimide (VIII) nach folgendem Schema herstellen:

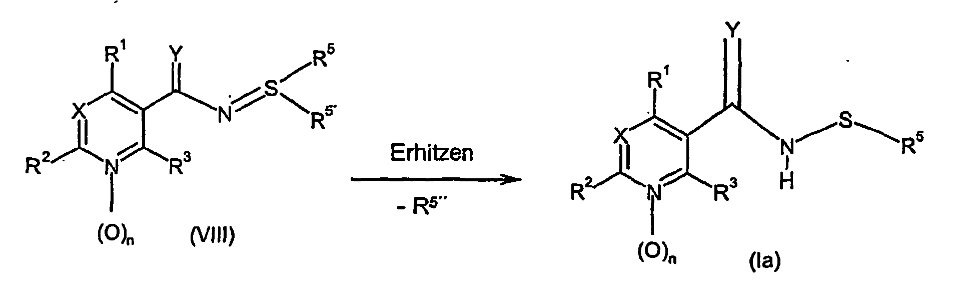

Dabei haben die Reste R¹, R², R³, R⁵, X und Y sowie der Index n die weiter oben angegebene Bedeutung, R^{5'} besitzt unabhängig von R⁵ eine der für R⁵ angegebenen Bedeutungen, und R^{5''} ist die um den Gehalt eines Wasserstoffatoms verminderte und von R^{5'} abgeleitete ethylenisch ungesättigte Abgangsgruppe. Neben den symmetrisch substituierten Verbindungen der Formel VIII (R⁵ = R^{5'}) können auch unsymmetrisch substituierte Verbindungen (R⁵ ≠ R^{5'}) zum Einsatz kommen.

Die Reaktion kann aus der reinen Substanz der Formel (VIII) bei Temperaturen über deren Schmelzpunkt durchgeführt werden. Es sind aber auch Umsetzungen in nichtprotischen organischen Lösungsmitteln möglich. So lässt sich beispielsweise S,S-2-Butyl-N-(4-trifluormethyl)-nicotinoyl-sulfimid durch 5-stündiges Erhitzen auf 100°C in N-2-Butylthio-4-trifluormethylnicotinamid überführen. Die Aufarbeitung kann nach Standardverfahren erfolgen, beispielsweise durch chromatographische Verfahren.

Die Umkehrung dieser Reaktion kann auch als Zugang zu den Sulfimiden der Formel (VIII) dienen. Die Erfindung betrifft daher auch ein Verfahren zur Herstellung von Verbindungen der Formel (VIII) durch Umsetzung von Verbindungen der Formel Ia. Zur Herstellung von Sulfimiden der allgemeinen Formel (VIII), in welchen R¹, R², R³, R⁵, Y, n und X die weiter oben zur Formel (I) angegebenen Bedeutungen haben, wird so verfahren, daß man eine Verbindung der allgemeinen Formel (la), worin R¹, R², R³, R⁵, Y, n und X die weiter oben angegebenen Bedeutungen haben, in Gegenwart einer Verbindung R⁵-Z, worin R⁵ die oben definierte Bedeutung aufweist und Z eine Abgangsgruppe ist, und einer Base gemäß nachfolgendem Schema umsetzt:

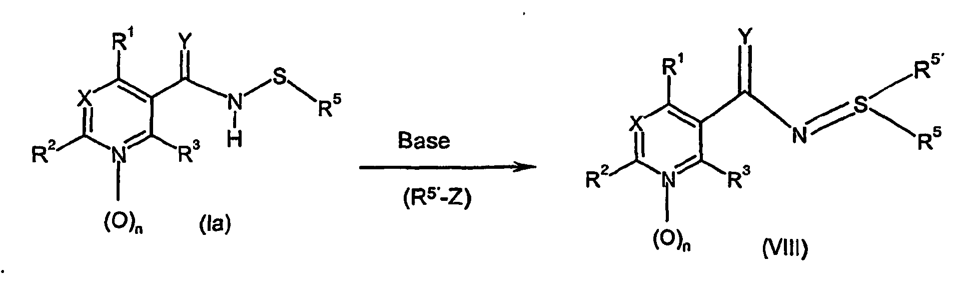

Typische Abgangsgruppen Z sind Halogen, Mesylat und Tosylat.

Neben den symmetrisch substituierten Verbindungen der Formel (VIII) (R⁵ = R^{5'}) können durch dieses Verfahren insbesondere auch die unsymmetrisch substituierten Verbindungen der Formel (VIII) (R⁵≠ R⁵) erhalten werden.

Das erfindungsgemäße Verfahren eignet sich vorzugsweise zur parallelisierten Reaktionsdurchführung.

Die Vorprodukte der Formel (VIII) für die Synthese der Verbindungen der Formel (I) lassen sich beispielsweise erhalten durch Umsetzung einer Carbonsäure oder Thiocarbonsäure der Formel (VI) 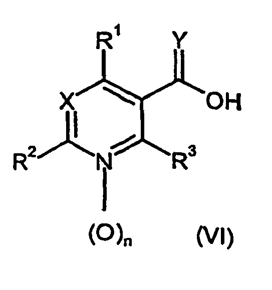 in der R¹, R², R³, X, Y und n die zur Formel (I) angegebenen Bedeutungen haben, in Form eines aktivierten Derivats dieser Säure, in Gegenwart einer Base, mit einer Verbindung der Formel (IX), vorzugsweise als Salz, 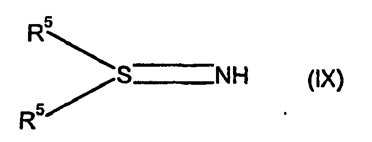 worin R⁵ die zur Formel (I) angegebenen Bedeutungen hat.

Die Derivatisierung der Verbindungen der Formel (Ib) zu Verbindungen der Formel (II) mit Resten R⁴ ungleich Wasserstoff über eine Mitsunobu Reaktion erfolgt gemäß folgendem Schema:

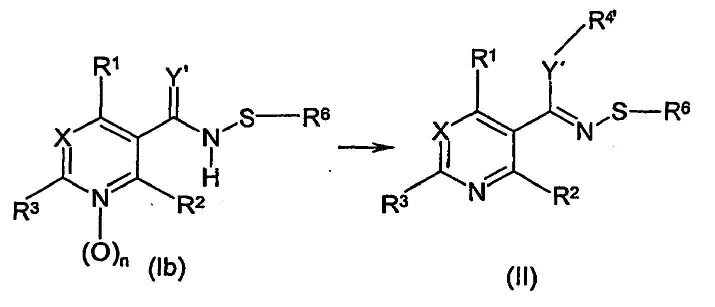

Dabei entstehen die O- bzw. S- derivatisierten Produkte der Formel (II) neben den N-derivatisierten Produkten der Formel (I) als Nebenprodukt.

Die Reaktion erfolgt unter Mitsunobu-Bedingungen, also durch Umsetzung des NH-Derivats mit einem Alkohol, R^{4'}-OH (z.B. Butanol), in Gegenwart eines Azodicarbonsäurediesters sowie eines Phosphins.

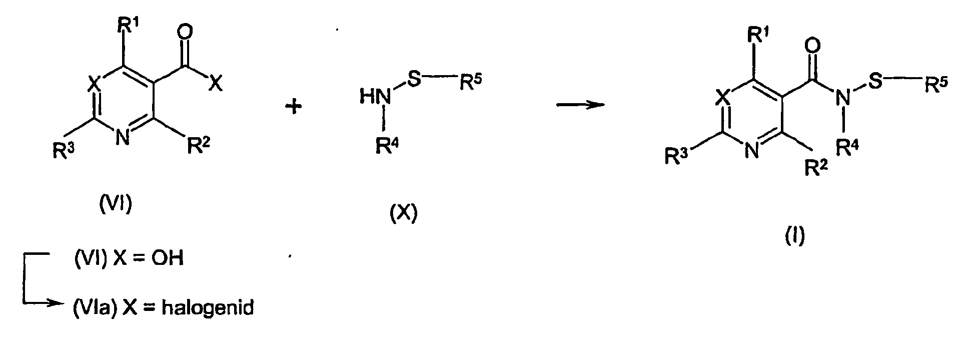

Für die Aktivierung der Säure (VI X = OH) kann die Umsetzung zum Säurehalogenid (VIa, X = halogenid) benutzt werden. Mögliche Halogenierungsreagenzien sind beispielsweise Oxalylchlorid, POCl₃, PCl₃, PCl₅, SOCl₂ oder SO₂Cl₂. Die so erhaltenen Säurehalogenide können dann weiter mit Thioaminen (X) umgesetzt werden. Als typische Methode wird das Säurehalogenid (VIa, X = halogenid) mit Thioaminen in Gegenwart einer Base umgesetzt. Geeignete Basen sind z.B. Alkali-oder Erdalkalimetall-hydroxide, - carbonate oder -hydrogencarbonate oder organische Basen, wie z.B. Trialkylamine oder Pyridine. Eingesetzt werden können auch Festphasen-gebundene Basen wie z.B. S-Trisamine von Agilent oder Polystyrene AM NH2 von Rapp. Die Reaktion wird vorteilhaft in einem inerten Lösemittel wie z.B. Dichlormethan, Chloroform, Tetrachlorkohlenstoff, Benzol, Toluol, Diethylether oder Tetrahydrofuran oder auch in Gemischen dieser Lösemittel in einem Temperaturbereich zwischen 0°C und 100°C, bevorzugt 20 °C und 50 °C durchgeführt.

Alternativ kann die Säure auch direkt unter Zuhilfenahme von Kupplungsreagenzien wie CDI, DCC oder EDAC mit Thioaminderivaten umgesetzt werden.

Zur Herstellung von Verbindungen der Formel (I) und (II), in denen n 1 bedeutet, kann, vorzugsweise vor Einführung der R⁵S-Gruppe, am Pyridin-Stickstoff oxidiert werden (siehe z.B. Houben-Weyl, Methoden der Organischen Chemie, Bd E 7b, Teil 2, S. 565, G. Thieme Verlag, Stuttgart 1992). Geeignete Oxidationsmittel sind beispielsweise organische Persäuren, wie 3-Chlorperbenzoesäure, und H₂O₂.

Die nach den obigen Verfahren hergestellten Verbindungen der Formel (I) und (II) können gegebenenfalls am Schwefel oxidiert werden (siehe z.B. Houben-Weyl. Methoden der Organischen Chemie, Bd E11, S.1299 ff., G. Thieme Verlag, Stuttgart 1985). Geeignete Oxidationsmittel sind beispielsweise Natriumperjodat oder organische Persäuren, wie 3-Chlorperbenzoesäure.

Weiterhin können gegebenenfalls Verbindungen der Formel (I) und (II), für die R² und/oder R³ ein Halogenatom, bevorzugt Chlor oder Fluor bedeuten, durch Umsetzung mit Alkoholen, Thiolen oder primären oder sekundären Aminen in Gegenwart einer Base In andere Verbindungen der Formel (I) und (II) überführt werden, in denen der Rest R² und/oder R³ eine Alkoxy, Alkylthio- oder Aminogruppe bedeutet.

Weitere Hinweise zu Herstellung der erfindungsgemäßen Verbindungen und der Verschiedenen Ausgangsprodukte finden sich In Standardwerken zur Organischen Synthese, wie z. B.: T. L. Gilchrist, C.J. Moody, Chem. Rev. 77, 409 (1977) oder Houben-Weyl, Methoden der Organischen Chemie, Bd. E11, S.877.

Kollektionen aus Verbindungen der Formel (I) und (II), die nach oben genannten Schema synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren, Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe Road, Tollesbury, Essex, CM9 8SE, England oder H+P Labortechnik GmbH, Bruckmannring 28, 85764 Oberschleißheim, Deutschland oder der Firma Radleys, Shirehill, Saffron Walden, Essex, England, angeboten werden. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen kann die Herstellung von Verbindungen der allgemeinen Formel (I) und (II) vollständig oder partiell durch Festphasen - unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen - unterstützte Synthesemethoden sind in der Fachliteratur hinreichend beschrieben, z.B. Barry A. Bunin in "The Combinatorial Index", Verlag Academic Press, 1998.

Die Verwendung von Festphasen-unterstützten Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (1) und (II) in Form von Substanzkollektionen, die Bibliotheken genannt werden.

Gegenstand der vorliegenden Erfindung sind auch Bibliotheken, die mindestens zwei Verbindungen der Formel (I) und/oder (II) enthalten.

Die Verbindungen der Formel (I) und (II) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats-und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:

Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp..

Aus der Ordnung der Isopoda z.B. Oniscus aselus, Armadium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung des Isoptera z.B. Reticulitermes spp..

Aus der Ordnung der Anoplura z.B. Phylloera vastatrix, Pemphigus spp., Pediculus humanus corporis, Haematopinus spp., Linognathus spp..

Aus der Ordnung der Mallophaga z.B. Trichodectes pp., Damalinea spp..

Aus der Ordnung der Thysanoptera z.B. Hercinothrips femoratis, Thrips tabaci.

Aus der Ordnung der Heteroptera z.B. Eurygaster spp., Dysdercus intermedius, Piesma quadrata, Cimex lectularius, Rhodnius prolixus, Triatoma spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopsis, Ceratophyllus spp..

Aus der Ordnung der Arachnida z.B. Scorpio maurus, Latrodectus mactans.

Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.

Aus der Klasse der Gastropoda z.B. Deroceras spp., Arion spp., Lymnaea spp., Galba spp., Succinea spp., Biomphalaria spp., Bulinus spp., Oncomelania spp.. Aus der Klasse der Bivalva z.B. Dreissena spp..
Weiterhin lassen sich Protozoen, wie Eimeria, bekämpfen.

Zu den pflanzenparasitären Nematoden, die erfindungsgemäß bekämpft werden können, gehören beispielsweise die wurzelparasitären Bodennematoden wie z.B. solche der Gattungen Meloidogyne (Wurzelgallennematoden, wie Meloidogyne incognita, Meloidogyne hapla und Meloidogyne javanica), Heterodera und Globodera (zystenbüdende Nematoden, wie Globodera rostochiensis, Globodera pallida, Heterodera trifolii) sowie der Gattungen Radopholus wie Radopholus similis, Pratylenchus wie Pratyglenchus neglectus, Pratylenchus penetrans und Pratylenchus curvitatus; Tylenchulus wie Tylenchulus semipenetrans, Tylenchorhynchus, wie Tylenchorhynchus dubius und Tylenchorhynchus claytoni, Rotylenchus wie Rotylenchus robustus, Heliocotylenchus wie Haliocotylenchus multicinctus, Belonoaimus wie Belonoaimus longicaudatus, Longidorus wie Longidorus elongatus, Trichodorus wie Trichodorus primitivus und Xiphinema wie Xiphinema index.

Ferner lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, beispielsweise Pflanzenschutzmittel, vorzugsweise insektizide, akarizide, ixodizide, nematizide, molluskizide oder fungizide, besonders bevorzugt insektizide und akarizide Mittel, die eine oder mehrere Verbindungen der Formel (I) und/oder (II) neben geeigneten Formulierungshilfsmitteln enthalten.

Die erfindungsgemäßen Mittel enthalten die Wirkstoffe der Formel (I) und/oder (II) im allgemeinen zu 1 bis 95 Gew.-%.

Zur Herstellung der erfindungsgemäßen Mittel gibt man den Wirkstoff und die weiteren Zusätze zusammen und bringt sie in eine geeignete Anwendungsform.

Die Erfindung betrifft auch Mittel, insbesondere insektizide und akarizide Mittel, die die Verbindungen der Formel (I) und/oder (II) neben geeigneten Formulierungshilfsmitteln enthalten.

Die erfindungsgemäßen Mittel enthalten die Wirkstoffe der Formeln (I) und/oder (II) im allgemeinen zu 1 bis 95 Gew.%. Sie können auf verschiedene Art formuliert werden, je nachdem wie es durch die biologischen und/oder chemischphysikalischen Parameter vorgegeben ist. Als Formulierungsmöglichkeiten kommen daher beispielsweise in Frage:

Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in: Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, d.h. Träger- und/oder oberflächenaktive Stoffe, wie Inertmaterialien, Tenside, Lösungsmittel und weitere Zusatzstoffe sind ebenfalls bekannt und beispielsweise beschrieben in: Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCuteheon's, "Detergents and Emulsifiers Annual", MC Publ. Corp., Ridgewood N.J.; Sisley and Wood, "Encyclopedia of Surface Active Agents", Chem. Publ. Co. lnc., N.Y. 1964; Schönfeldt, "Grenzflächenaktive Äthylenoxidaddukte", Wiss. Verlagsgesell., Stuttgart 1967; Winnacker-Küchier, "Chemische Technologie", Band 7, C. Hanser Verlag Miinchen, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Cadodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise gewünschtenfalls in Mischung mit Düngemitteln - granuliert werden.

In Spritzpulvern beträgt die Wirkstoffkonzentration üblicherweise etwa 10 bis 90 Gew.-%, der Rest zu 100 Gew.% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kg/ha oder mehr Aktivsubstanz, vorzugsweise liegt sie jedoch zwischen 0,001 und 5 kg/ha Wirkstoff.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:
1. aus der Gruppe der Phosphorverbindungen
   Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Flupyrazofos, Fonofos, Formothion, Fosthiazate, Heptenophos, Isazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;
2. aus der Gruppe der Carbamate
   Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morphotinothio)carbamate (UC 51717), Triazamate;
3. aus der Gruppe der Carbonsäureester
   Acrinathrin, Allethrin, Alphametrin, 5-Benzyl-3-furylmethyl-(E)-(1R)-cis-2,2-dimethyl-3-(2-oxothiolan-3-ylidenemethyl)cyclopropanecarboxylate, Beta-Cyfluthrin, alpha-Cypermethrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1RS)-trans-3-(4-tert.butylphenyl)-2,2-dimethylcyclopropanecarboxylate (NCl 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flubrocythrinate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin, Tralomethrin, Transfluthrin, Zeta-Cypermethrin (F-56701);
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, ABG-9008, Acequinocyl, Azadirachtin, Acetamiprid, Anagrapha falcitera, AKD-1022, AKD-3059, AKD-3088, AL-9811, ANS-118, Bacillus thuringiensis, Beauveria bassianea, Bensultap, Bifenazate (D-2341), Binapacryl, BJL-932, Bromopropylate, BAJ-2740 (Spirodiclofen), BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chloproxyfen, Clothianidine, Chromafenozide (ANS-118), A-184699, Clothianidine, Cyclopropancarbonsäure-(2-naphthylmethyl)ester (Ro12-0470), Cyromazin, CM-002X, DBI-3204, Diacloden (Thiamethoxam), Diafenthiuron, DBI-3204, N-(3,5-Dichlor-4-(1,1,2,3,3,3-hexafluor-1-propyloxy)phenyl)-carbamoyl)-2-chlorbenzcarboximid-säureethylester, DDT, Dicofol, Diflubenzuron, N-(2,3-Dihydro-3-methyl-1,3-thiazol-2-ylidene)-2,4-xylidine, Dihydroxymethyl-dihydroxypyrrolidin, Dinobuton, Dinocap, Diofenolan, DPX-062, Emamectin-Benzoate (MK-244), Endosulfan, Ethiprole (Sulfethiprole), Ethofenprox, Etoxazole (Y1-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granulose- und Kernpolyederviren, Fenpyroximate, Fenthiocarb, Fluacrypyrim, Flufenzine, Flubenzimine, Flubrocythrinate, Flucycloxuron, Flufenoxuron, Flufenprox (ICI-A5683), Flufenzine, Fluproxyfen, FMC-F6028, Gamma-HCH, Halofenozide (RH-0345), Halofenprox (MTI-732), Hexaflumuron (DE_473), Hexythiazox, HOI-9004, Hydramethylnon (AC 217300), Lufenuron, L-14165, Imidacloprid, Indoxacarb (DPX-MP062), Kanemite (AKD-2023), M-020, MTI-446, Ivermectin, M-020, IKA-2000, IKI-220 (Flonicamid), MKI-245, Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Novaluron, NC-196, NNI-0001, Nidintefuran, Propargite, Pyriproxyfen (S-71639), Pirydaryl, Protrifenbute, Pyriproxyfen, NC-196, NC-1111, NNI-9768, Novaluron (MCW-275), OK-9701, OK-9601, OK-9602, OK-9802, Propargite, Pymethrozine, Pyridaben, Pyrimidifen (SU-8801), R-195, RH-0345, RH-2485, RYI-210, S-1283, S-1833, SB7242, SI-8601, Silafluofen, Silomadine (CG-177), Spinosad, Spirodiclofen, SU-9118, Tebufenozide, Tebufenpyrad (MK-239), Teflubenzuron, Tetradifon, Tetrasul, Thiacloprid, Thiocyclam, Thiamethoxam, TI-435, Tolfenpyrad (OMI-88), Triazamate (RH-7988), Triflumuron, Triethoxyspinosyn A, Verbutin, Vertalec (Mykotal), YI-5301 und Yi-6101.

Die oben genannten Kombinationspartner stellen bekannte Wirkstoffe dar, die zum großen Teil in Ch.R Worthing, S.B. Walker, The Pesticide Manual, 12. Auflage, British Crop Protection Council Famham, 2000 beschrieben sind.

Als Fungizide, die mit den erfindungsgemäßen Verbindungen der Formel (I) und/oder (II) kombiniert werden können, sind z.B. folgende Produkte zu nennen:

Aldimorph, Andoprim, Anilazine, BAS 480F, BAS 450F, Benalaxyl, Benodanil, Benomyl, Binapacryl, Bitertanol, Bromuconazole, Buthiobate, Captafol, Captan, Carbendazim, Carboxin, CGA 173506, Cyprofuram, Dichlofluanid, Dichlomezin, Diclobutrazole, Diethofencarb, Difenconazole (CGA 169374), Difluconazole, Dimethirimol, Dimethomorph, Diniconazole, Dinocap, Dithianon, Dodemorph, Dodine, Edifenfos, Ethirimol, Etridiazot, Fenarimol, Fenfuram, Fenpicionil, Fenpropidin, Fenpropimorph, Fentinacetate, Fentinhydroxide, Ferimzone (TF164), Fluazinam, Fluobenzimine, Fluquinconazole, Fluorimide, Flusilazole, Flutolanil, Fluthafol, Folpet, Fosetylaluminium, Fuberidazole, Fulsulfamide (MT-F 651), Furalaxyl, Furconazole, Furmecyclox, Guazatine, Hexaconazole, ICI A5504, Imazalil, Imibenconazole, Iprobenfos, Iprodione, Isoprothiolane, KNF 317, Kupferverbindungen wie Cu-oxychlorid, Oxine-Cu, Cu-oxide, Mancozeb, Maneb, Mepanipyrim (KIF 3535), Metconazole, Mepronil, Metalaxyl, Methasulfocarb, Methfuroxam. MON 24000, Myclobutanil, Nabam, Nitrothalido-propyl, Nuarimol, Ofurace, Oxadixyl, Oxycarboxin, Penconazole, Pencycuron, PP 969, Probenazole, Propineb, Prochloraz, Procymidon, Propamocarb, Propiconazole, Prothiocarb, Pyracarbolid, Pyrazophos, Pyrifenox, Pyroquilon, Rabenzazole, RH7592, Schwefel, Tebuconazole, TF 167, Thiabendazole, Thicyofen, Thiofanate-methyl, Thiram, Tolclofos-methyl, Tolylfluanid, Triadimefon, Triadimenol, Tricyclazole, Tridemorph, Triflumizol, Triforine, Validamycin, Vinchlozolin, XRD 563, Zineb, Natriumdodecylsulfonat, Natrium-dodecyl-sulfat, Natrium-C13/C15-alkoholethersulfonat, Natriumcetostearyl-phosphatester, Dioctyl-natrium-sulfosuccinat, Natrium-isopropylnaphthalene-sulfonat, Natrium-methylenebisnaphthalene-sulfonat, Cetyl-trimethylammonium-chlorid, Salze von langkettigen primären, sekundären oder tertiären Aminen, Alkyl-propyleneamine, Lauryl-pyrimidiniumbromid, ethoxylierte quartemierte Fettamine, Alkyl-dimethyl-benzyl-ammoniumchlorid und 1-Hydroxyethyl-2-alkyl-imidazolin.

Der Wirkstoffgehalt der aus den handelsüblichen Formulierungen bereiteten Anwendungsformen kann von 0,00000001 bis zu 95 Gew.% Wirkstoff, vorzugsweise zwischen 0,00001 und 1 Gew.% liegen.

Die Anwendung geschieht in einer den Anwendungsformen angepaßten üblichen Weise.

Gegenstand der Erfindung sind daher auch die Verwendung der Verbindungen der Formel (I) und/oder (II) oder ihrer Salze zur Bekämpfung von tierischen Schädlingen und ein Verfahren zur Bekämpfung von tierischen Schädlingen, wobei man die tierischen Schädlinge direkt oder indirekt in Kontakt mit einer Verbindung der Formel (I) oder (II) oder deren Salzen bringt.

Die erfindungsgemäßen Wirkstoffe eignen sich auch zur Bekämpfung von Endo-und Ektoparasiten auf dem human- und veterinärmedizinischen Gebiet bzw. auf dem Gebiet der Tierhaltung. Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpudems sowie durch parenterale Anwendung in Form beispielsweise der Injektion.

Die erfindungsgemäßen Verbindungen der Formel (1) und (II) können demgemäß auch besonders vorteilhaft zur Behandlung von Warmblütlern, insbesondere in der Viehhaltung (z.B. Rinder, Schafe, Schweine und Geflügel wie Hühner, Gänse usw.) eingesetzt werden. In einer bevorzugten Ausführungsform der Erfindung werden den Tieren die Verbindungen, gegebenenfalls in geeigneten Formulierungen und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Neben den bisher genannten Applikationsverfahren zeigen die erfindungsgemäßen Wirkstoffe der Formel (I) und (II) eine hervorragende systemische Wirkung. Die Wirkstoffe können daher auch über Pflanzenteile, unterirdische wie oberirdische (z.B. Wurzel, Stolonen, Stengel, Stamm, Blatt), in die Pflanzen eingebracht werden, wenn die Wirkstoffe in flüssiger oder fester Form auf, in und/oder in die direkte Umgebung der Pflanze appliziert werden (z.B. Granulate in der Erdapplikation, Applikation in gefluteten Reisfeldern, Stamminjektion bei Bäumen, Stengelbandagen bei perenierenden Pflanzen).

Daneben sind die erfindungsgemäßen Wirkstoffe, die gegebenfalls in Coformulierung mit Fungiziden in besonderer Weise zur Behandlung von vegetativem und generativem pflanzlichem Vermehrungsmaterial einsetzbar, wie z.B. von Saatgut von beispielsweise Getreide, Gemüse, Baumwolle, Reis, Zuckerrübe und anderen Kultur- und Zierpflanzen, von Zwiebeln, Stecklingen und Knollen weiterer vegetativ vermehrter Kultur und Zierpflanzen. Die Behandlung hierfür kann vor der Saat bzw. dem Pflanzvorgang erfolgen (z.B. durch spezielle Techniken des 'Seed Coatings', durch Beizung in flüssiger oder fester Form oder als 'Seedbox Treatment'), während des Saatvorgangs bzw. des Pflanzens oder nach dem Saat- bzw. Pflanzvorgang durch spezielle Applikationstechniken (z.B. Saatreihenbehandlung). Die angewandte Wirkstoffmenge kann entsprechend der Anwendung in einem größerem Bereich schwanken. Im allgemeinen liegen die Aufwandmengen zwischen 1 g und 10 kg Wirkstoff pro Hektar Bodenfläche. Die Behandlungsverfahren für pflanzliches Vermehrungsmaterial und das so behandelte pflanzliche Vermehrungsmaterial sind weitere Gegenstände der Erfindung.

Die Verbindungen der Formel (I) und (II) können auch zur Bekämpfung von tierischen Schädlingen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten, wie bestimmten Insekten oder Mikroorganismen, wie Pilzen, Bakterien oder Viren. Andere besondere Eigenschaften betreffen z.B. das Erntegut hinsichtlich Menge, Qualität, Lagerfähigkeit, Zusammensetzung und spezieller lnhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung in wirtschaftlich bedeutenden transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten.

Bei der Anwendung in transgenen Kulturen, insbesondere mit Insektenresistenzen treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Schädlingsspektrum, das bekämpft werden kann oder veränderte Aufwandmengen, die für die Applikation eingesetzt werden können.

Gegenstand der Erfindung ist deshalb auch die Verwendung von Verbindungen der Formel (I) und (II) zur Bekämpfung von Schadorganismen, insbesondere tierischen Schädlingen, in transgenen Kulturpflanzen.

Neben lethaler Wirkung auf Schädlingen zeichnen sich die Verbindungen der Formel (I) und (II) auch durch einen ausgeprägten Repellenteffekt aus.

Repellent im Sinne der Verbindung ist ein Stoff oder Stoffgemisch, das abwehrend oder vertreibend auf andere Lebewesen, insbesondere Schädlinge und Lästlinge wirkt. Der Begriff umfaßt dabei auch Effekte wie den Antifeeding-Effekt, wobei die Nahrungsaufnahme gestört oder verhindert wird (fraßabweisender Effekt), Unterdrückung der Eiablage oder eine Beeinflussung der Populationsentwicklung.

Gegenstand der Erfindung ist daher auch die Verwendung von Verbindungen der Formel (I) und (II) zur Erzielung der genannten Effekte, insbesondere bei den in den biologischen Beispielen benannten Schädlingen.

Gegenstand der Erfindung ist auch ein Verfahren zur Abwehr oder Vertreibung von Schadorganismen, wobei man eine oder mehrere Verbindungen der Formel (I) und/oder (II) an dem Ort ausbringt, von dem die Schadorganismen ferngehalten oder vertrieben werden sollen.

Ausbringen kann im Falle einer Pflanze beispielsweise eine Behandlung der Pflanze aber auch des Saatguts bedeuten.

Es ist, was die Beeinflussung von Populationen angeht von Interesse, daß die Effekte auch hintereinander bei der Entwicklung einer Population beobachtet werden, wobei sie sich aufaddieren können. Hierbei kann der Einzeieffekt selbst nur einen Wirkungsgrad deutlich unter 100 %ige haben und insgesamt am Ende doch eine 100 % Wirkung erreicht werden.

Außerdem zeichnen sich die Verbindungen der Formel (I) und (II) dadurch aus, daß man - will man die oben angeführten Effekte ausnutzen - zu einem früheren Zeitpunkt als bei einer direkten Bekämpfung üblich das Mittel appliziert. Der Effekt hält häufig lange Zeit an, so daß eine Wirkungsdauer von mehr als 2 Monaten erreicht wird.

Die Effekte treten bei Insekten, Spinnentieren und den anderen der oben erwähnten Schädlinge auf.

Die Anwendung der erfindungsgemäßen Verbindungen beinhaltet neben direkter Applikation auf die Schädlinge jede andere Applikation, bei der Verbindungen der Formel (I) und/oder (II) auf die Schädlinge wirken. Solche indirekten Applikationen können beispielsweise in der Anwendung von Verbindungen liegen, die, beispielsweise im Boden, der Pflanze oder dem Schädling, zu Verbindungen der Formel (I) und/oder (II) zerfallen oder abgebaut werden.

Auf den Inhalt der deutschen Patentanmeldung 101 468 73.3, deren Priorität die vorliegende Anmeldung beansprucht, sowie auf den Inhalt der beiliegenden Zusammenfassung wird hiermit ausdrücklich Bezug genommen; sie gelten durch Zitat als Bestandteil dieser Beschreibung.

Die nachfolgenden Beispiele dienen zur Erläuterung der Erfindung.

### I. Chemische Beispiele

### Beispiel A: Herstellung von N-Cyclohexylthio-(4-trifluormethyl)nicotinamid

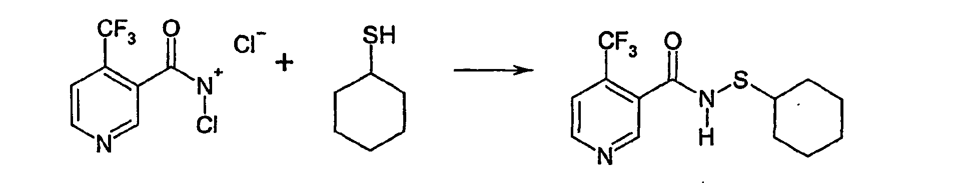

Cyclohexylmercaptan (1 g = 0,009 mol) und Triethylamin (1,2 ml = 0,009 mol) wurden in 20 ml Acetonitril vorgelegt und anschließend die überstehende Lösung aus N-Chlor-4-trifluormethyl-nikotinsäureamid-hydrochlorid (2,24 g = 0,009 mol) in Triethylamin (1,2 ml = 0,009 mol) in 10 ml Acetonitril dazugetropft. Die Mischung wurde drei Stunden bei Raumtemperatur gerührt. Zur Aufarbeitung wurde das Acetonitril abdestilliert, der Rückstand mit Aceton ausgerührt und der Niederschlag abgesaugt. Nach Waschen mit Aceton wurden die organischen Phasen vereinigt, das Lösungsmittel entfernt und das verbleibene Öl über Chromatographie an Kieselgel mit Ethylacetat gereinigt. 1,64 g (63%) N-Cyclohexylthio-(4-trifluormethyl)nicotinamid wurden als helles Öl erhalten.

### Beispiel B: Herstellung von N-(2-Butylthio)-(4-trifluormethyl)nicotinamid

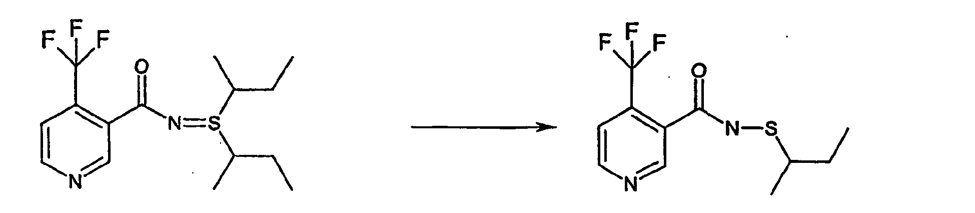

S,S-2-Butyl-N-(4-trifluormethyl)nicotinoyl sulfimid (0,5 g = 0,0015 mol) wurde 5 Stunden auf 100 °C erhitzt. Die Reaktionsmischung wurde in Ethylacetat aufgenommen und über einen Kieselgelpad filtriert. Nach Abziehen des Lösungsmittels verbleiben 0,26 g (62,5 %) N-(2-Butylthio)-(4-trifluormethyl)nicotinamid als farbloses Öl (nmr (DMSO): 9.75 s 1H; 8.95 d 6 Hz 1H, 8.84 s 1H, 7.86 d 6 Hz 1 H, 3.00 m 1H. 1.60 m 1H, 1.44 m 1 H, 1.20 d 7 Hz 3H, 0.96 tr 7 Hz 1H).

### Beispiel C: Herstellung von N-(Isopropyl)-(4-trifluormethyl)nicotinamid und von Verbindung (X)

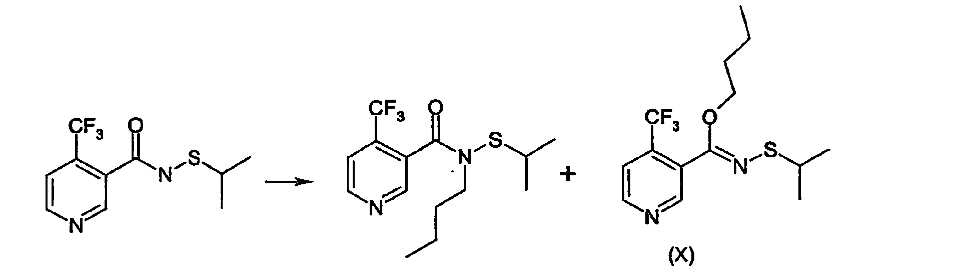

3,95 g (0,015 mol) Triphenylphosphin wurden in 100 ml THF gelöst. Unter 5 °C wurden 2,35 ml (0,015 mol) Azodicarbonsäurediethylester zugetropft, die Lösung dann 20 Minuten bei Raumtemperatur gerührt. Dann 1,35 ml 1-Butanol zugetropft und bei Raumtemperatur gertihrt. Nach 5 Minuten wurde eine Lösung aus 2,65 g N-lsopropylthio-(4-trifluormethyl)nicotinamid in 25 ml THF dazugegeben und 20 Stunden bei Raumtemperatur gerührt. Die Substanzmischung wurde dann eingeengt und über eine Säule mit n-Heptan : Ethylacetat 1:1 gereinigt. 0,17 g (5 %) N-Butyl-N-isopropylthio-(4-trifluormethyl)nicotinamid und 1,2 g (37 %) Verbindung (X) wurden als Öle isoliert.

### Beispiel D: Herstellung von N-Benzylthio-N-cyclohexyl-(4-trifluormethyl)-nicotinamid

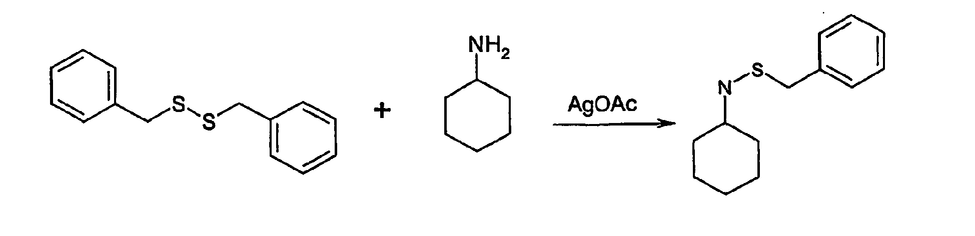

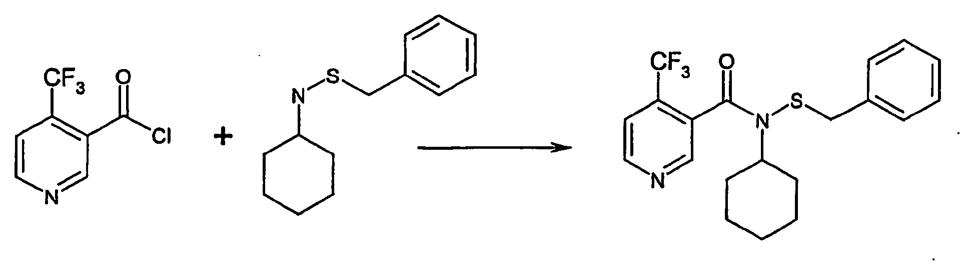

0,81 g (0,0033 mol) Dibenzyldisulfid und 1,1 g (0,0066 mol) Silberacetat wurden in 40 ml Ethylacetat vorgelegt, auf 0-5°C gekühlt und 1,9 ml (0,016 mol) Cyclohexylamin dann zugetropft. Die Reaktion war leicht exotherm. Das Eisbad wurde entfernt und der Kolben mit Alufolie umwickelt und über Nacht bei Raumtemperatur gerührt. Die Reaktion wurde dann filtriert, das Lösungsmittel entfernt und die Reaktionsmischung in Diethylether und Wasser aufgenommen sowie vom Ungelösten abfiltriert. Die organische Phase wurde mit Wasser gewaschen, getrocknet und das Lösungsmittel dann entfernt. Man isolierte als Wachs 0,15 g (18,5%) des Produktes N-(Cyclohexyl)-N-(benzylthio)amin.

0,14 g (0,0006 mol) N-Cyclohexyl-N-(benzylthio)amin und 0,16 ml (0,0011 mol) Triethylamin wurden in 2,5 ml THF gelöst und 0,22 g (0,001 mol) (4-Trifluormethyl)-nikotinsäurechlorid dann zugetropft. Die Reaktionsmischung wurde 4 Stunden bei Raumtemperatur gerührt. Zur Aufarbeitung wurden Ethylacetat und Wasser dazugegeben, die organische Phase eingeengt und dann an Kieselgel mit Ethylacetat: n-Heptan 2:3 chromatographiert. Man erhielt 0,12 g (51%) N-Benzylthio-N-cyclohexyl-(4-trifluormethyl)nicotinamid.

In analoger Weise werden die in den nachfolgenden Tabellen aufgeführten Verbindungen hergestellt.

**Tabelle 1**

| | | | | |
|---|---|---|---|---|
| 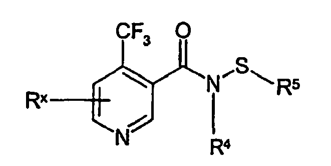 | | | | |

| Beispiel Nr. | R^{x} | R⁴ | R⁵ | F.P. (°C) |
|---|---|---|---|---|
| 1 | | H | cyclopentyl | Öl |
| 2 | | H | phenyl | Öl |
| 3 | | ethyl | phenyl | Öl |
| 4 | | 2-propyl | phenyl | Öl |
| 5 | | H | 4-methylphenyl | |
| 6 | | 2-propyl | 4-methylphenyl | Öl |
| 7 | | H | 2-bromophenyl | |
| 8 | | H | pentafluorophenyl | |
| 9 | | H | 2- chlorophenyl | |
| 10 | | H | 2,5 - dichlorophenyl | |
| 11 | | H | 2,6 - dichlorophenyl | |
| 12 | | ethyl | 2,6 - dichlorophenyl | |
| 13 | | methyl | 2,6 - dichlorophenyl | |
| 14 | | H | 2-methoxyphenyl | |
| 15 | | H | 2-isopropyphenyl | |
| 16 | | H | 2-methylphenyl | |
| 17 | | H | 3- chlorophenyl | |
| 18 | | H | 4-chlorophenyl | 120 |
| 19 | | H | 3,4- dichlorophenyl | |
| 20 | | H | 3-methoxyphenyl | |
| 21 | | H | 3-methylphenyl | |
| 22 | | H | 4-bromophenyl | |
| 23 | | H | 4-fluorophenyl | 106 |
| 24 | | H | 4- chlorophenyl | |
| 25 | | H | 4-acetamidophenyl | |
| 26 | | H | 4-methoxyphenyl | |
| 27 | | H | t-butyl | |
| 28 | | H | t-amyl | |
| 29 | | H | 2-propyl | 86 |
| 30 | | H | methyl | |
| 31 | | H | benzyl | |
| 32 | | H | 2-chloro-benzyl | |
| 33 | | H | 4-chloro-benzyl | |
| 34 | | H | 4-methoxy-benzyl | |
| 35 | | H | methyl-2-acetyl | |
| 36 | | H | ethyl-2-acetyl | |
| 37 | | H | 2-methyl-1-propyl | |
| 38 | | H | 2-methyl-1-butyl | Öl |
| 39 | | cyclohexyl | 2-methyl-1-butyl | |
| 40 | | H | 1-dodecyl | |
| 41 | | H | 1-nonyl | |
| 42 | | H | ethyl | |
| 43 | | H | 2 - phenyl ethyl | |
| 44 | | H | allyl | Öl |
| 45 | | H | 3-methyl-1-butyl | Öl |
| 46 | | H | propyl | |
| 47 | | H | 1-butyl | |
| 48 | | H | 1-pentyl | Öl |
| 49 | | H | 1- hexyl | Öl |
| 50 | | H | 1- heptyl | Öl |
| 51 | | H | 1 - octyl | |
| 52 | | H | 1 - nonyl | |
| 53 | | H | 2- imidazoyl | |
| 54 | | H | 1- methyl - 2- imidazoyl | |
| 55 | | . H | 2- benzimidazoyl | |
| 56 | | H | 2- benzoxazoyl | |
| 57 | | H | 6-ethoxy-2-benzoxazoyl | |
| 58 | | H | 5-chloro-2-benzoxazoyl | |
| 59 | | H | 3,4,5,6-tetrahydropyrimidyl | |
| 60 | | H | 2-pyrimidyl | |
| 61 | | H | 4,6-dimethyl-2-pyrimidyl | |
| 62 | | H | 2-pyridyl | |
| 63 | | H | 4-pyridyl | |
| 64 | | H | 7-trifluoromethyl-4-quinolinyl | |
| 65 | | H | 2,6 -dimethylphenyl | |
| 66 | | H | 2-ethyl-phenyl | |
| 67 | | H | 2-methyl- 3-furyl | |
| 68 | | H | 4-phenyl-2-thiazoyl | |
| 69 | | H | 4-t-butyl-phenyl | |
| 70 | | H | N-(4-methyl-phenyl)acetamide | |
| 71 | | H | 4-aza-2-benzimidazoyl | |
| 72 | | H | 2 (5-methyl-1,3,4 thiadiazoyl) | |
| 73 | | H | 2,4-dichlorobenzyl | |
| 74 | | H | 3,4-dichlorobenzyl | |
| 75 | | H | 4-methyl-benzyl | |
| 76 | | H | 3-fluoro-phenyl | |
| 77 | | H | 1,3,4-thiadiazoyl | |
| 78 | | H | 4-fluoro-benzyl | |
| 79 | | H | 3-(trifluoromethyl)phenyl | |
| 80 | | H | 2-fluorophenyl | |
| 81 | | H | 2,4-dichlorophenyl | |
| 82 | | H | 3,5-dichlorophenyl | |
| 83 | | H | 4-1-propylphenyl | |
| 84 | | H | 4-trifluoromethylbenzyl | 119 |
| 85 | | H | 3-trifluoromethylbenzyl | Öl |
| 86 | | H | 2-pydidylmethyt | |
| 87 | | H | 4-trifluoromethoxybenzyl | |
| 88 | | H | 2-chloro- 6-fluorobenzyl | |
| 89 | | H | 3-(4-chlorophenyl)-1,2,4 triazoyl | |
| 90 | | H | 3,4-dimethoxyphenyl | |
| 91 | | H | 3-butanoyl | |
| 92 | | H | 5-phenyl-1,3,4-oxadiazoyl | |
| 93 | | H | 2-thienylmethyl | Öl |
| 94 | | H | 4-trifluoromethylpyri-2-yl | |
| 95 | | H | 3-chloro-5-trifluoromethyl-pydir2-yl | |
| 96 | | H | dimethylaminoethyl | |
| 97 | | H | 3-tritluoromethylpyrid-2-yl | |
| 98 | | H | cyclopropyl | Öl |
| 99 | | H | morpholln-4-ylmethyl | |
| 100 | | H | 4-pyrimidyl | |
| 101 | | H | 5-methyl-4H-1,2,4-triazoyl | |
| 102 | | H | cyclobutyl | |
| 103 | | 4-fluoro-phenyl | cyclobutyl | |
| 104 | | benzyl | cyclobutyl | |
| 105 | | methyl | cyclobutyl | |
| 106 | | ethyl | cyclobutyl | |
| 107 | | 2-propyl | cyclobutyl | |
| 108 | | cyclohexyl | cyclobutyl | |
| 109 | | butyl | cyclobutyl | |
| 110 | 2,6-dichloro | H | 2-nitrophenyl | 240 |
| 111 | | H | hex-1-en-5-yl | |
| 112 | | H | 4-chloro-phenyl | |
| 113 | | H | 2,5-dimethylphenyl | |
| 114 | | H | 2-(methoxycarbonyl)ethyl | |
| 115 | | H | 2-(trimethylsilyl)ethyl | Öl |
| 116 | | H | 2-furylmethyl | Öl |

**Tabelle 2**

| | | | | |
|---|---|---|---|---|
| 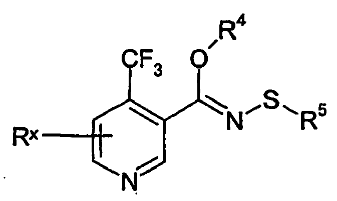 | | | | |

| Beispiel Nr. | R^{x} | R⁴ | R⁵ | mp (°C) |
|---|---|---|---|---|
| 117 | | But-1-yl | Isopropyl | |
| 118 | | Prop-1-yl | Isopropyl | |
| 119 | | 1-Methyl-prop-1-yl | Isopropyl | |
| 120 | | 2-Vinyloxy-eth-1-yl | Isopropyl | |
| 121 | | Pent-4-en-1-yl | Isopropyl | |
| 122 | | 4-Methyloxy-but-1-yl | Isopropyl | |
| 123 | | 4-Methylthio-but-1-yl | Isopropyl | |
| 124 | | 3-Nitro-but-1-yl | Isopropyl | |

### B. Formulierungsbeispiele

a) Ein Stäubemittel wird erhalten, indem man 10 Gew;-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobemsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

In den folgenden Beispielen 1 und 2 werden Verbindungen als aktiv angesehen, wenn sie bei einer Konzentration von 500 ppm (bezogen auf den Gehalt an Wirkstoff) oder weniger eine Wirkung auf die Schadorganismen von 50% oder mehr aufweisen.

### Beispiel 1

Angekeimte Ackerbohnen-Samen (Vicia faba) mit Keimwurzeln wurden in mit Leitungswasser gefüllte Braunglasfläschen übertragen und anschließend mit ca. 100 schwarzen Bohnenblattläusen (Aphis fabae) belegt. Pflanzen und Blattläuse wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25°C, 40-60% RF). Nach 3 und 6 Tagen Lagerung wurde die Mortalität des Präparates auf die Blattläuse festgestellt. Die Verbindungen gemäß der folgenden Beispiele waren aktiv: A, B, C,1, 2, 3, 4, 6, 18, 23, 29, 38, 44, 45, 48, 49, 50, 84, 85, 98, 115, 116, 124.

### Beispiel 2

Angekeimte Ackerbohnen-Samen (Vicia faba) mit Keimwurzeln wurden in mit Leitungswasser gefüllte Braunglasfläschen übertragen. Vier Milliliter einer wäßrigen Lösung des zu prüfenden und formulierten Präparates wurden in das Braunglasfläschen hineinpipettiert. Anschließend wurde die Ackerbohne mit ca. 100 schwarzen Bohnenblattläusen (Aphis fabae) stark belegt. Pflanzen und Blattläuse wurden dann in einer Klimakammer gelagert (16 Stunden Licht/Tag, 25°C, 40-60 % RF). Nach 3 und 6 Tagen Lagerung wurde die wurzelsystemische Wirkung des Präparates als Mortalität der Blattläuse festgestellt. Die Verbindungen gemäß der folgenden Beispiele waren aktiv: A, B, C, 1, 2, 3, 4, 6, 18, 23, 29, 38, 44, 45, 48, 49, 50, 84, 85, 98, 115, 116, 124.

## Patentansprüche

1. Verbindungen der Formel (I) und deren Salze, 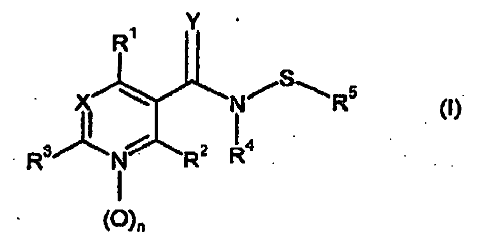 wobei die Symbole und Indizes folgende Bedeutungen haben:
X ist =CH- oder N-;
Y ist =O oder =S;
n ist 0 oder 1;
R¹ ist (C₁-C₆)-Alkyl, (C₁-C₆)-Haloalkyl, -S(Halogcn)s oder Halogen, wobei eine oder zwei CH₂-Gruppen durch -O- oder -S- oder N(C₁-C₆)-Alkyl ersetzt sein können, mit der Maßgabe, daß Heteroatome nicht benachbart sein dürfen;
R², R³ sind unabhängig voneinander Wasserstoff, (C₁-C₆)-Alkyl, (C₁-C₆)-Haloalkyl oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
R⁴ ist Wasserstoff (C₁-C₁₀)-Alkyl, (C₃-C₁₀)-Cycloalkyl, (C₃-C₁₀)-Alkenyl, (C₃-C₁₀)-Alkinyl, (C₆-C₁₄)-Aryl, (C₃-C₁₀)-Heterocyclyl oder (C₁-C₁₀)-Alkanoyl, wobei die genannten Reste gegebenenfalls ein- oder mehrfach substituiert sein können;
R⁵ ist Wasserstoff, (C₁-C₂₀)-Alkyl (C₃-C₁₀)-Alkenyl, (C₃-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl, wobei die genannten Reste gegebenenfalls ein- oder mehrfach substituiert sein können;
wobei Verbindungen der Formel (I), worin X =CH und R⁵ gegebenenfalls substituiertes (C₁-C₁₀)-Alkyl bedeuten, ausgeschlossen sind.

2. Verbindungen der Formel (II) und deren Salze, 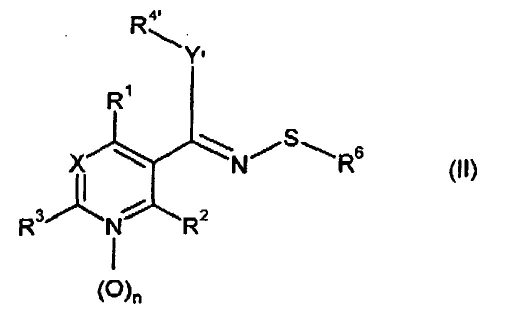 wobei die Symbole und Indizes folgende Bedeutungen haben:
X ist =CH- oder =N-;
Y' ist -O- oder -S-;
n ist 0 oder 1 ;
R¹ ist (C₁-C₆)-Alkyl, (C₁-C₆)-Haloalkyl, -S(Halogen)₅ oder Halogen, wobei eine oder zwei CH₂-Gruppen durch -O- oder -S- oder -N(C₁-C₆)-Alkyl ersetzt sein können, mit der Maßgabe, daß Heteroatome nicht-benachbart sein dürfen;
R², R³ sind unabhängig voneinander Wasserstoff, (C₁-C₆)-Alkyl, (C₁-C₆)-Haloalkyl oder Halogen, wobei eine oder zwei CH₂-Gruppen durch -O- oder -S- oder -N(C₁-C₆)-Alkyl ersetzt sein können, mit der Maßgabe, daß Heteroatome nicht benachbart sein dürfen;
R⁴ ist Wasserstoff, (C₁-C₁₀)-Alkyl, (C₃-C₁₀)-Cycloalkyl, (C₃-C₁₀)-Alkenyl, (C₃-C₁₀)-Alkinyl, (C₆-C₁₄)-Aryl oder (C₃-C₁₀)-Heterocyclyl, wobei die genannten Reste gegebenenfalls ein- oder mehrfach substituiert sein können; und
R⁶ ist Wasserstoff, (C₁-C₁₀)-Alkyl, (C₃-C₁₀)-Alkenyl, (C₃-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl, wobei die genannten Reste gegebenenfalls ein- oder mehrfach substituiert sein können.

3. Verbindungen der Formel (I) und deren Salze nach Anspruch 1, worin R¹ SF₅, CHF₂, CF₂Cl oder CF₃ ist.

4. Verbindungen der Formel (I) und deren Salze nach Anspruch 1 oder 3, worin R² und R³ Wasserstoff sind.

5. Verbindungen der Formel (1) und deren Salze nach einem oder mehreren der Ansprüche 1, 3 oder 4, worin X ==CH- ist, Y =O bedeutet, n 0 darstellt, R¹ CF₃ bedeutet, R², R³ und R⁴ Wasser-stoff sind und R⁵ (C₁-C₁₀)-Alkyl, (C₂-C₁₀)-Alkenyl, (C₂-C₁₀)-Alkinyl, (C₃-C₈)-Cycloalkyl, (C₄-C₈)-Cycloalkenyl, (C₈-C₁₀)-Cycloalkinyl, Aryl oder Heterocyclyl ist, wobei die genannten Reste gegebenenfalls ein- oder mehrfach substituiert sein können.

6. Verbindungen der Formel (1) und deren Salze nach einem oder mehreren der Ansprüche 1, 3, 4 oder 5, worin R⁵ einen Rest der Formel IIa darstellt, 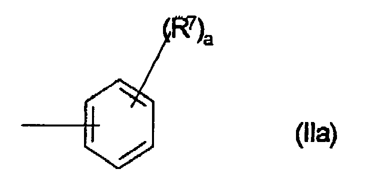 wobei die Symbole und Indizes folgende Bedeutungen haben:
a ist 0, 1, 2, 3 oder 4;
R⁷ ist gleich oder verschieden R⁸ oder zwei Reste R⁷ bilden zusammen mit den Atomen, an die sie gebunden sind, ein drei- bis achtgliedriges, gesättigtes oder ungesättigtes, gegebenenfalls mit einem oder mehreren Resten R⁸ substituiertes Ringsystem, das gegebenenfalls auch weitere Heteroatome aus der Gruppe O, N, S, SO und SO₂, enthält;
R⁸ ist gleich oder verschieden R⁹, R¹⁰, -C(W)R⁹, -C(=NOR⁹)R⁹, -C(=NNR⁹₂)R⁹_{,} -C(=W)OR⁹, -C(=W)NR⁹₂, -OC(=W)R⁹, -OC(=W)OR⁹, -NR⁹C(=W)R⁹, -N[C(=W)R⁹]₂, -NR⁹C(=W)OR⁹, -C(=W)NR⁹-NR₂, -C(=W)NR⁹-NR⁹[C(=W)R⁹], -NR⁹-C(--W)NR⁹₂, -NR⁹-NR⁹C(=W)R⁹, -NR⁹-N[C(=W)R⁹]₂,-N[(C=W)R⁹]-NR⁹₂, -NR⁹ N[(C=W)WR⁹], -NR⁹[(C=W)NR⁹₂], -NR⁹(C=NR⁹)R⁹, -NR⁹(C=NR⁹)NR⁹₂, -O-NR⁹₂, -O-NR⁹(C=W)R⁹, -SO₂NR⁹₂,-NR⁹SO₂R⁹, -SO₂OR⁹, -OSO,₂R⁹, -OR⁹, -NR⁹₂, -SR⁹, -SiR⁹₃, -PR⁹₂, -P(=W)R⁹₂, -SOR⁹, -SO₂R⁹, -PW₂R⁹₂, -PW₃R⁹₂ oder zwei Reste R⁸ sind zusammen (=W). (=N-R⁹), (= CR₂⁹, (= CHR) oder (=CH₂);
W ist = o oder = s;
R⁹ ist gleich oder verschieden (C₁-C₆)-Alkyl, (C₂-C₆)-Alkenyl, (C₂-C₆)-Alkinyl, (C₃-C₈)-Cycloalkyl, (C₄-C₈)-Cycloalkenyl, (C₃-C₈)-Cycloallcyl-(C₁-C₄)-Alkyl, (C₄-C₈)-Cycloalkenyl-(C₁-C₄)-Alkyl, (C₃-C₈)-Cycloalkyl-(C₂-C₄)-Alkenyl, (C₄-C₈)-Cycloalkenyl-(C₂-C₄)-Alkenyl, (C₁-C₆)-Alkyl-(C₃-C₈)-Cycloalkyl, (C₂-C₆)-Alkcnyl-(C₃-C₈)-Gycloalkyl, (C₂-C₆)-Alkinyl-(C₃-C₈)-Cycloalkyl, (C₁-C₆)-Alkyl-(C₄-C₈)-Cycloalkenyl, (C₂-C₆)-Alkenyl-(C₄-C₈)-Cycloalkenyl, Aryl, Heterocyclyl;
wobei die genannten Reste gegebenenfalls mit einem oder mehreren Resten R¹⁰ substituiert sind und gegebenenfalls zwei Reste R⁹ zusammen ein Ringsystem bilden;
R¹⁰ ist gleich oder verschieden Halogen, Cyano, Nitro, Hydroxy, Thio, Amino, Formyl, (C₁-C₆)-Alkanoyl, (C₁-C₆)-Alkoxy, (C₃-C₆)-Alkenyloxy, (C₃-C₆)-Alki-nyloxy, (C₁-C₆)-Haloalkyloxy, (C₃-C₆)-Haloalkenyloxy, (C₃-C₆)-Haloalkinyl-oxy, (C₃-C₈)-Cycloalkoxy, (C₄-C₈)-Cyclealkenyloxy, (C₃-C₈)-Halocycloalk-oxy, (C₄-C₈)-Halocycloalkenyloxy, (C₃-C₈)-Cycloalkyl-(C₁-C₄)-Alkoxy, (C₄-C₈)-Cycloalkenyl-(C₁-C₄)-Alkoxy, (C₃-C₈)-Cycloalkyl-(C₂-C₄)-Alkenyloxy, (C₄-C₈)-Cycloalkenyl-(C₁-C₄)-Alkenyloxy, (C₁-C₆)-Alkyl-(C₃-C₈)-Cycloalk-oxy, (C₂-C₆)-Alkenyl -(C₃-C₅)-Cycloalkoxy, (C₂-C₆)-Alkinyl-(C₃-C₈)-Cycloalk-oxy, (C₁-C₆)-Alkyl-(C₄-C₈)-Cycloalkenyloxy. (C₂-C₆)-Alkenyl-(C₄-C₈)-Cyclo-alkenyloxy, (C₁-C₄)-Alkoxy-(C₁-C₆)-Alkoxy, (C₁-C₄)-Alkoxy-(C₃-C₆)-Alke-nyloxy, Carbamoyl, (C₁-C₆)-Mono- oder Dialkylcarbamoyl, (C₁-C₆)-Mono-oder -Dihaloalkylearbamoyl, (C₃-C₈)-Mono- oder -Dieycloatkylcarhasnoyl, (C₁-C₆)-Alkoxycarbonyl, (C₃-C₈)-Cycloalkoxycarbonyl, (C₁-C₆)-Alkanoyloxy, (C₃-C₈)-Cycloalkanoyloxy, (C₁-C₆)-Haloalkoxycarbonyl, (C₁-C₆)-Haloalkano-yloxy, (C₁-C₆)-Alkanoylamino, (C₁-C₆)-Haloalkanoylamino, (C₂-C₆)-Alkenoylamino, (C₃-C₈)-Cycloalkanoylxmino, (C₃-C₈)-Cycloallcyl-, (C₁-C₄)-Alkanoylamino, (C₁-C₆)-Alkylthio, (C₃-C₆)-Alkenylthio, (C₃-C₆)-Alkinylthio, (C₁-C₆)-Haloalkylthio, (C₃-C₆)-Haloalkenylthio. (C₃-C₆)-Haloalkinylthio, (C₃-C₈)-Cycloalkylthio, (C₄-C₈)-Gycloalkenylthio, (C₃-C₈)-Halocycloalkthio, (C₄-C₈)-Halocycloalkenylthio, (C₃-C₈)-Cycloalkyl-(C₁-C₄)-Alkylthio, (C₄-C₈)-Cycloalkenyl-(C₁-C₄)-Alkylthio, (C₃-C₈)-Cycloalkyl-( C₃-C₄)-Alkenylthio, (C₄-C₈)-Cycloalkenyl-(C₃-C₄)-Alkenylthio, (C₁-C₆)-Alkyl-(C₃-C₈)-Cycloalkylthio, (C₂-C₆)-Alkenyl-(C₃-C₈)-Cycloalkylthio, (C₂-C₆)-Alkinyl-(C₃-Cₛ)-Cycloalkylthio, (C₁-C₆)-Alkyl-(C₄-C₈)-Cycloalkenylthio, (C₂-C₆)-Alkenyl-(C₄-C₈)-Cycloalkenylthio, (C₁-C₆)-Alkylsulfinyl, (C₃-C₆)-Alkenylsulflnyl, (C₃-C₆)-Alkinylsulfinyl, (C₁-G₆)-Haloalkylsafinyl, (C₃-C₆)-Haloalkenylsulfinyl, (C₃-C₆)-Haloalkinylsulfinyl, (C₃-C₈)-Cycloalkylsulfinyl, (C₄-C₈)-Cycloalkenylsulfinyl, (C₃-C₈)-Halocycloalkylsulfinyl, (C₄-C₈)-Halocycloallcenylsulfinyl, (C₃-C₈)-Cycloalkyl(C₁-C₄)-Alkylsulfinyl, (C₄-C₈)-Cycloalkenyl-(C₁-C₄)-Alkylsulfinyl, (C₃-C₈)-Cycloalkyl-(C₃-C₄)-Alkenylsulfinyl, (C₄-C₈)-Cycloalkenyl-( C₃-C₄)-Alkenylsulfinyl, (C₁-C₆)-Alkyl-(C₃-C₈)-Cycloalkylsulfinyl, (C₂-C₆)-Alkenyl-(C₃-C₈)-Cycloalkylsulfinyl, (C₂-C₆)-Alkinyl-(C₃-C₈)-Cycloalkylsulfinyl, (C₁-C₆)-Alkyl-(C₄-C₈)-Cycloalkenylsulfinyl, (C₂-C₆)-Alkenyl-(C₄-C₈)-Cycloalkenylsulfinyl, (C₁-C₆)-Alkylsulfonyl, (C₃-C₆)-Alkenylsulfonyl, (C₃-C₆)-Alkinyisulfonyl, (C₁-C₆)-Haloalkylsulfonyl, (C₃ *-C₆)-* HaloalkenylsuJfony I, (C₃-C₆)-Haloalkinylsulfonyl, (C₃-C₈)-Cycloalkylsulfonyl, (C₄-C₈)-Cycloalkenylsulfonyl, (C₃-C₈)-Halocycloalkylsulfonyl, (C₄-C₅)- Halocycloalkenylsulfonyl, (C₃-C₈)-Cycloalkyl-(C₁-C₄)-Alkylsulfonyl, (C₄-C_{R})-Cycloalkenyl-(Cᵢ-C₄)-Alkylsulfonyl, (C₃-C₈)-Cycloalkyl-(C₃-C₄)-Alkenylsulfonyl, (C₄-C₈)-Cycloalkenyl-(C₃-C₄)-Alkenylsulfonyl, (C₁ -C₆)-Alkyl-(C₃-C₈)-Cycloalkylsulfony , (C₂-C₆)-Alkenyl-(C₃-C₈)-Cycloalkylsulfonyl, (C₂-C₆)-Alkinyl-(C₃-Cₓ)-Cycloalkylsulfonyl, (C₁-C₆)-Alkyl-(C₄-C₈)-Cycloalkenylsulfonyl, (C₂-C₆)-Alkenyl-(C₄-C₈)-Cycloalkenylsulfonyl, (C₁-C₆)-Dialkylamino, (C₁-C₆)-Alkylamino, (C₃-C₆)-Alkenylamino, (C₃-C₆)-Alkinylamino, (C₁-C₆)-Haloalkylamino, (C₃-C₆)-Haloalkenylamino, (C₃-C₆)-Haloalkinylamino, (C₃-C₈)-Cycloalkylamino, (C₄-C₈)-Cycloalkenylamino, (C₃-C₈)-Halocycloalkamino, (C₄-C₈)-Halocycloalkenylamino, (C₃-C₈)-Cycloalkyl-(C₁-C₄)-Alkylamino, (C₄-C₈)-Cycloalkenyl-( C₁-C₄)-Alkylamino. (C₃-C₈)-Cycloalkyl-(C₃-C₄)-Alkenylamino, (C₄-C₈),Cycloalkenyl-(C₃-C₄)-Alkenylamino, (C₁-C₆)-Alkyl-(C₃-C₈)-Cycloalkylamino, (C₂-C₆)-Alkenyl-(C₃-C₈)-Cycloalkylamino, (C₂-C₆)-Alkinyl-(C₃-C₈)-Cycloalkylamino, (C₁-C₆)-Alkyl-(C₄-C₈)-Cycloalkenylamino, (C₂-C₆)-Alkenyl-(C₄-C₈)-Cycloalkenylamino, (C₁-C₆)-Trialkylsilyl, Aryl, Aryloxy, Arylthio, Arylsulfinyl, Arylsulfonyl, Arylamino, Aryl-(C₁-C₄)-Alkoxy, Aryl-(C₃-C₄)-Alkenyloxy, Aryl-(C₁-C₄)-Alkylthio, Aryl-(C₁-C₄)-Alkylsulfinyl, Aryl-(C₁-C₄)-Alkylsulfonyl, Aryl-(C₂-C₄)-Alkenylthio, Aryl-(C₂-C₄)-Alkenylsulfinyl, Aryl-(C₂-C₄)-Alkenylsulfonyl, Aryl-(C₁-C₄)-Alkylamino, Aryl-(C₃-C₄)-Alkenylamino, Aryl-(C₁-C₆)-Diaikylsilyl, Diaryl-(C₁-C₆)-Alkylsilyl, Triarylsilyl und 5- oder 6-gliedriges Heterocyclyl, wobei der cyclische Teil der vierzehn letztgenannten Reste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkyl-amino, (C₁-C₄)-Haloalkylamino und (C₁-C₄)-Alkanoyl substituiert ist, und, falls R⁹ Aryl oder Heterocyclyl ist, (C₁-C₄)-Alkyl oder (C₁-C₄)-Haloalkyl.

7. Verbindungen der Formel (11) und deren Salze nach Anspruch 2, worin R¹ SF₅, CHF₂, CF₂Cl oder CF₃ ist.

8. Verbindungen der Formel (11) und deren Salze nach Anspruch 2 oder 7, worin X =CH- ist, Y'-O- bedeutet, n 0 darstellt, R¹ CF₃ bedeutet, R², R³ und R^{4'} Wasserstoff sind und R⁶ (C₁-C₁₀)-Alkyl, (C₃-C₈)-Cycloalkyl, Aryl, Benzyl oder Heterocyclyl mit insgesamt ein bis drei Stickstoff-, Sauerstoff- und/oder Schwefelringatomen bedeutet, wobei die genannten Reste gegebenenfalls ein- oder mehrfach substituiert sein können.

9. Verbindungen der Formel (II) und deren Salze nach Anspruch 2, 7 oder 8, worin als Substituenten an den Resten R^{4'} und R⁶ die Gruppen R⁷ mit folgender Bedeutung vorliegen: R⁷ ist gleich oder verschieden R⁸ oder zwei Reste R⁷ bilden zusammen mit den Atomen, an die sie gebunden sind, ein drei- bis achtgliedriges, gesättigtes oder ungesättigtes, gegebenenfalls mit einem oder mehreren Resten R⁸ substituiertes Ringsystem, das gegebenenfalls auch weitere Heteroatome, aus der Gruppe O, N, S, SO und SO₂, enthält; und R⁸ besitzt die in Anspruch 6 definierte Bedeutung.

10. Verfahren zur Herstellung von Verbindungen der Formel. (I) nach einem oder mehreren der Ansprüche 1.3,4,5 und 6 umfassend die Schritte: 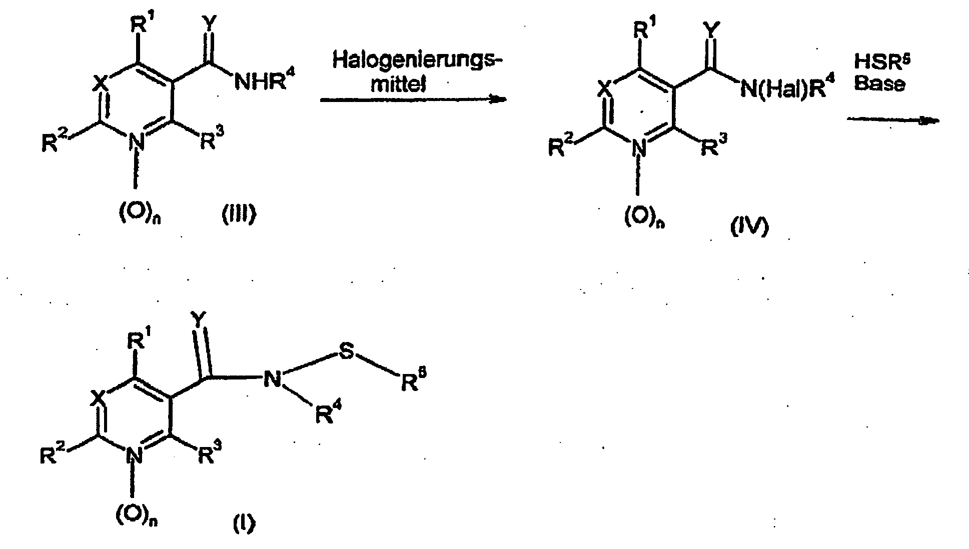
a) Umsetzung eines Carbonsäureamids der Formel (III) mit einem Halogenierungsmittel zu einer Verbindung der Formel (IV), und
b) Umsetzung dieser Verbindung mit einem Thioether R⁵SH in Gegenwart einer Base zu den Endprodukten der Formel (I) , worin in diesen Formeln die Resto R¹, R², R³, R⁴. R⁵, X und Y sowie Index n die in Anspruch 1 angegebenen Bedeutungen haben und Hal Halogen bedeutet

11. Verfahren zur Herstellung von Verbindungen der Formel (I) nach einem oder mehreren der Ansprüche 1, 3, 4, 5 und 6 umfassend die Umsetzung eines aktivierten Derivats der Carbonsäure oder Thiocarbonsäure der Formel (VI) 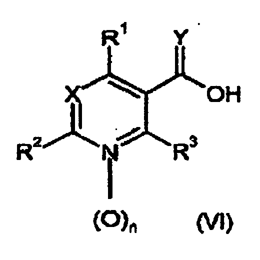 in Gegenwart einer Base mit einer Verbindung der Formel (VII), 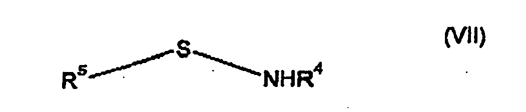 worin in diesen Formeln die Reste R¹, R², R³, R⁴, R⁵, X und Y sowie die in Anspruch 1 angegebenen Bedeutungen haben.

12. Verfahren zur Herstellung von Verbindungen der Formel (Ia) nach Anspruch 1 durch thermische Zersetzung der Sulfimide der Formel (VIII) 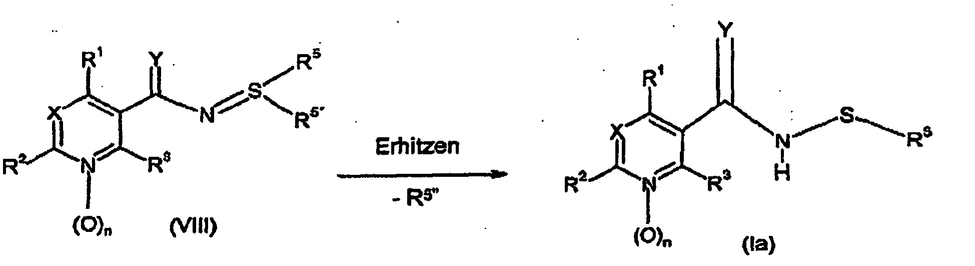 worin die Reste R¹, R², R³, R⁵, X und Y sowie der Index n die in Anspruch 1 definierte Bedeutung besitzen, R^{5'} eine der in Anspruch 1 für R⁵ definierten Gruppen darstellt, die ein β-Wasserstoffatom aufweist, und R^{5"} die um den Gehalt eines Wasserstoffatoms verminderte, zu R⁵' korrespondierende ethylenisch ungesättigte Abgangsgruppe darstellt.

13. Verfahren zur Herstellung von Verbindungen der Formel (II) nach einem oder mehreren der Ansprüche 2, 7, 8 und 9 umfassend die Umsetzung der Verbindungen der Formel (Ib), 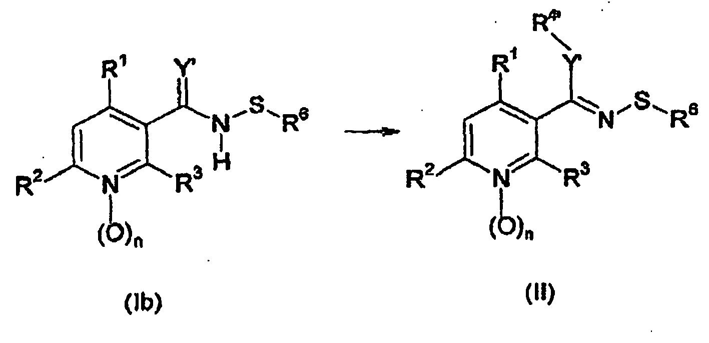 mit einem Alkohol R⁴'-OH in Gegenwart eines Azodicarbonsäurediesters sowie eines Phosphins gemäß obigem Schema zu den Verbindungen_der Formel (II), worin R⁴' eine der in Anspruch 2 definierten Bedeutungen außer H besitzt und R¹, R², R³, R⁶, X, Y' und n eine der in Anspruch 2 definierten Bedeutungen aufweisen.

14. Mittel mit insektizider, akarizider, ixodizider, nematizider, molluskizider und/oder fungizider Wirkung **gekennzeichnet durch** einen Gehalt an mindestens einer Verbin= dung der Formel (I) und/oder (II) oder eines ihrer Salze nach Anspruch 1 und/oder 2.

15. Verwendung der Verbindungen der Formel (I) und/oder (II) oder ihrer Salze nach Anspruch 1 und/oder 2 zur Herstellung einer Zusammensetzung oder eines Mittels zur Bekämpfung von tierischen Schädlingen.

16. Verwendung der Verbindungen der Formel (I) und/oder **(II)** oder ihrer Salze nach Anspruch 1 und/oder 2 zur Herstellung einer Zusammensetzung oder eines Mittels zur Abwehr oder Vertreibung von Schädlingen und/oder Lästlingen.

17. Verwendung der Verbindungen der Formel (I) und/oder (II) oder ihrer Salze nach Anspruch 1 und/oder 2 zur Herstellung eines Tierarzneimittels.

18. Verfahren zur Bekämpfung von tierischen Schädlingen, wobei man die tierischen Schädlinge direkt oder indirekt in Kontakt mit einer Verbindung der Formel (I) oder (II) oder deren Salzen gemäß Anspruch 1 oder 2 bringt, ausgenommen Verwendungen bzw. Verfahren am menschlichen oder tierischen Körper.

19. Verfahren zur Abwehr oder Vertreibung von Schadorganismen, wobei man eine oder mehrere Verbindungen der Formel (I) und /oder (II) oder deren Salze gemäß Anspruch 1 und/oder 2 an dem Ort ausbringt, von dem die Schadorganismen ferngehalten oder vertrieben werden sollen, ausgenommen Verwendungen bzw. Verfahren am menschlichen oder tierischen Körper.

20. Verfahren zur Herstellung von Sulfimiden der Formel (VIII) durch Umsetzung einer Thiohydroxamsäure der allgemeinen Formel (Ia) in Gegenwart einer Verbindung R^{5'}-Z und einer Base gemäß nachfolgendem Schema: 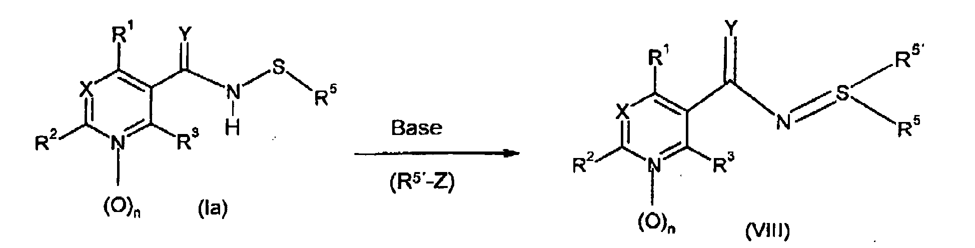 worin die Reste R¹, R², R³, R⁵, X und Y sowie der Index n die in Anspruch 1 definierte Bedeutungen besitzen, R^{5'} unabhängig von R⁵ eine der in Anspruch 1 für R⁵ definierten Gruppen darstellt, und Z eine Abgangsgruppe

## Claims

1. Compounds of formula (I) and salts thereof, 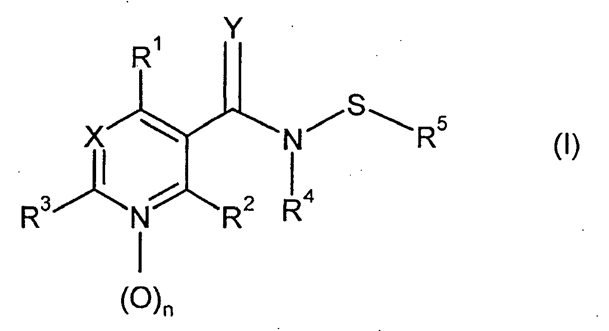 wherein the symbols and indices have the following meaning:
X is =CH- or =N-;
Y is =O or =S;
n is 0 or 1;
R¹ is (C₁-C₆)-alkyl, (C₁-C₆)-haloalkyl, S(halogen)₅ or halogen, wherein one or two CH₂-groups can be substituted by -O- or -S- or -N(C₁-C₆)-alkyl, provided that heteroatoms must not be adjacent;
R², R³ are, independent of each other, hydrogen, (C₁-C₆)-alkyl, (C₁-C₆)-haloalkyl or halogen, where one or two CH₂-groups can be substituted by -O- or -S- or -N(C₁-C₆)-alkyl, provided that heteroatoms must not be adjacent;
R⁴ is hydrogen, (C₁-C₁₀)-alkyl, (C₃-C₁₀)-cycloalkyl, (C₃-C₁₀)-alkenyl, (C₃-C₁₀)-alkinyl, (C₆-C₁₄)-aryl, (C₃-C₁₀)-heterocyclyl or (C₁-C₁₀)-alkanoyl,
wherein these groups can have one or more substituents;
R⁵ is hydrogen, (C₁-C₁₀)-alkyl, (C₃-C₁₀)-alkenyl, (C₃-C₁₀)-alkinyl, (C₃-C₈)-cycloalkyl, (C₄-C₈)-cycloalkenyl, (C₈-C₁₀)-cycloalkinyl, aryl or heterocyclyl, wherein these groups can have one or more substituents;
wherein compounds of formula (I) where X is =CH- and R⁵ is optionally substituted (C₁-C₁₀)-alkyl are excluded.

2. Compounds of formula (II) and salts thereof, 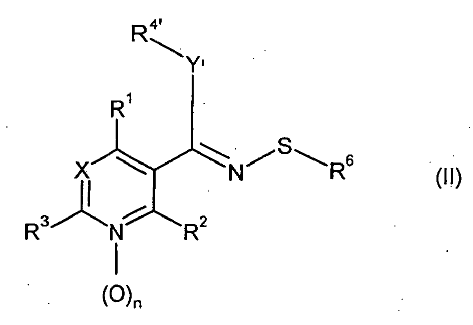 wherein the symbols and indices have the following meaning:
X is =CH- or =N-;
Y' is =O or =S;
n is 0 or 1;
R¹ is (C₁-C₆)-alkyl, (C₁-C₆)-haloalkyl, S(halogen)₅ or halogen, where one or two CH₂-groups can be substituted by -O- or -S- or -N(C₁-C₆)-alkyl provided that heteroatoms must not be adjacent;
R², R³ are, independent of each other, hydrogen, (C₁-C₆)-alkyl, (C₁-C₆)-haloalkyl or halogen, where one or two CH₂-groups can be substituted by -O- or -S- or -N(C₁-C₆)-alkyl provided that heteroatoms must not be adjacent;
R^{4'} is hydrogen, (C₁-C₁₀)-alkyl, (C₃-C₁₀)-cycloalkyl, (C₃-C₁₀)-alkenyl, (C₃-C₁₀)-alkinyl, (C₆-C₁₄)-aryl, (C₃-C₁₀)-heterocyclyl or (C₁-C₁₀)-alkanoyl,
wherein these groups can have one or more substituents;
R⁶ is hydrogen, (C₁-C₁₀)-alkyl, (C₃-C₁₀)-alkenyl, (C₃-C₁₀)-alkinyl, (C₃-C₈)-cycloalkyl, (C₄-C₈)-cycloalkenyl, (C₈-C₁₀)-cycloalkinyl, aryl or heterocyclyl, wherein these groups can have one or more substituents;

3. Compounds of formula (I) and salts thereof according to claim 1, wherein R¹ is SF₅, CHF₂, CF₂Cl or CF₃.

4. Compounds of formula (I) and salts thereof according to claim 1 or 3, wherein R² and R³ are hydrogen.

5. Compounds of formula (I) and salts thereof according to one or more of claims 1, 3 or 4, wherein X is =CH-, Y is =O, n is 0, R¹ is CF₃, R², R³ and R⁴ are hydrogen and R⁵ is (C₁-C₁₀)-alkyl, (C₂-C₁₀)-alkenyl, (C₂-C₁₀)-alkinyl, (C₃-C₈)-cycloalkyl, (C₄-C₈)-cydoalkenyl, (C₈-C₁₀)-cycloalkinyl, aryl or heterocyclyl, wherein these groups can have one or more substituents.

6. Compounds of formula (I) and salts thereof according to one or more of the claims 1, 3, 4, or 5, wherein R⁵ is a residue of formula (IIa), 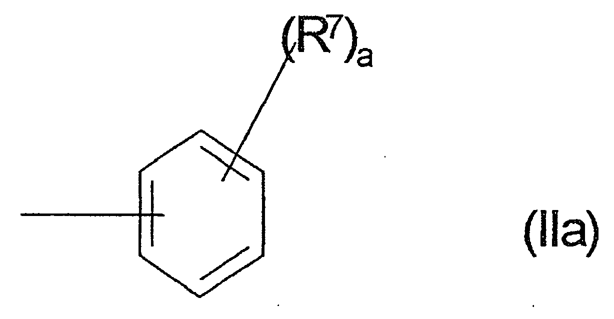 wherein the symbols and indices have the following meaning:
a is 0, 1 , 2, 3 or 4;
R⁷ is identical or different R⁸ or two residues R⁷ form, together with the atoms to which they are bound, a three- to eight-membered saturated or unsaturated ring system and which optionally is substituted with one or more residues R⁸ and optionally comprises additional heteroatoms selected from the group consisting of O, N, S, SO and SO₂
R⁸ is identical or different R⁹, R¹⁰, -C(W)R⁹, -C(=NOR⁹)R⁹, -C(=NNR⁹₂)R⁹, -C(=W)OR⁹, -C(=W)NR⁹₂, -OC(=W)R⁹, -OC(=W)OR⁹, -NR⁹C(=W)R⁹,-N[C(=W)R⁹]₂, -NR⁹C(=W)OR⁹, -C(=W)NR⁹-NR⁹₂, -C(=W)NR⁹-NR⁹[C(=W)R⁹], -NR⁹-C(=W)NR⁹₂, -NR⁹-NR⁹C(=W)R⁹, NR⁹-N[C(=W)R⁹]₂, -N[(C=W)R⁹]-NR⁹₂, -NR⁹N[(C=W)WR⁹],-N R⁹[(C=W)N R⁹_{2]}, -NR⁹(C=NR⁹)R⁹, -NR⁹(C=NR⁹)NR⁹₂, -O-NR⁹₂, -O-NR⁹(C=W)R⁹, -SO₂NR⁹₂ -NR⁹SO₂R⁹, -SO₂OR⁹, -OSO₂R⁹, -OR⁹,-NR⁹₂, -SR⁹, SiR⁹₃, -PR⁹₂, -P(=W)R⁹₂, -SOR⁹, -SO₂R⁹, -PW₂R⁹₂,-PW₃R⁹₂, or two residues R⁸ together are (=W), (=N-R⁹), (=CR⁹₂), (=CHR⁹) or (=CH₂);
W is =O or =S
R⁹ is identical or different (C₁-C₆)-alkyl, (C₂-C₆)-alkenyl, (C₂-C₆)-alkinyl, (C₃-C₈)-cycloalkyl, (C₄-C₈)-cycloalkenyl, (C₃-C₈)-cycloalkyl-(C₁-C₄)-alkyl, (C₄-C₈)-cycloalkenyl-(C₁-C₄)-alkyl, (C₃-C₈)cycloalkyl-(C₂-C₄)-alkenyl, (C₄-C₈)-cycloalkenyl-(C₂-C₄)-alkenyl, (C₁-C₆)-alkyl-(C₃-C₈)-cycloalkyl, (C₂-C₆)-alkenyl-(C₃-C₈)-cycloalkyl, (C₂-C₆)-alkinyl-(C₃-C₈)-cycloalkyl, (C₁-C₆)-alkyl-(C₄-C₈)-cycloalkenyl, (C₁-C₆)-alkenyl-(C₄-C₈)-cycloalkenyl, aryl, heterocyclyl;
wherein these residues are optionally substituted with one or more residues R¹⁰ and optionally two residues R⁹ together form a ring system;
R¹⁰ is identical or different halogen, cyano, nitro, hydroxyl, thio, amino, formyl, (C₁-C₆)-alkanoyl, (C₁-C₆)-alkoxy, (C₃-C₆)-alkenyloxy, (C₃-C₆)-alkinyloxy, (C₁-C₆)-haloalkyloxy, (C₃-C₆)-haloalkenyloxy, (C₃-C₆)-haloalkinyloxy, (C₃-C₈)-cycloalkoxy, (C₄-C₈)-cycloalkenyloxy, (C₃-Cₐ)-halocycloalkoxy, (C₄-C₈)-halocycloalkenyloxy, (C₃-C₈)-cycloalkyl-(C₁-C₄)-alkoxy, (C₄-C₈)-cydoalkenyl-(C₁-C₄)-alkoxy, (C₃-C₈)-cycloalkyl-(C₂-C₄)-alkenyloxy, (C₄-C₈)-cycloalkenyl-(C₁-C₄)-alkenyloxy, (C₁-C₆)-alkyl-(C₃-C₈)- cycloalkoxy, (C₂-C₆)-alkenyl-(C₃-C₈)-cycloalkoxy, (C₂-C₆)-alkinyl-(C₃-C₈)-cycloalkoxy, (C₁-C₆)-alkyl-(C₄-C₈)-cycloalkenyloxy, (C₂-C₆)-alkenyl-(C₄-C₈)-cycloalkenyloxy, (C₁-C₄)-alkoxy-(C₁-C₆)-alkoxy, (C₁-C₄)-alkoxy-(C₃-C₆)-alkenyloxy, carbamoyl, (C₁-C₆)-mono- or dialkylcarbamoyl, (C₁-C₆)-mono or -dihaloalkylcarbamoyl, (C₃-C₈)-mono or -dicycloalkylcarbamoyl, (C₁-C₆)-alkoxycarbonyl, (C₃-C₈)-cyclo-alkoxycarbonyl, (C₁-C₆)-alkanoyloxy, (C₃-C₈)-cycloalkanoyloxy, (C₁-C₆)-haloalkoxycarbonyl, (C₁-C₆)-haloalkanoyloxy, (C₁C₆)-alkanoyl-amino, (C₁-C₆)-haloalkanoylamino, (C₂-C₆)-alkenoylamino, (C₃-C₈)-cycloalkanoylamino, (C₃-C₈)-cycloalkyl, (C₁-C₄)-alkanoylamino, (C₁-C₆)-alkylthio, (C₃-C₆)-alkenylthio, (C₃-C₆)-alkinylthio, (C₁-C₆)-haloalkylthio, (C₃-C₆)-haloalkenylthio, (C₃-C₆)-haloalkinylthio, (C₃-C₈)-cycloalkylthio, (C₄-C₈)-cydoalkenylthio, (C₃-C₈)-halocycloalkylthio, (C₄-C₈)-halocydoalkenylthio, (C₃-C₈)-cycloalkyl-(C₁-C₄)-alkylthio, (C₄-C₈)-cycloalkenyl-(C₁-C₄)-alkylthio, (C₃-C₈)-cycloalkyl-(C₃-C₄)-alkenylthio, (C₄-C₈)-cycloalkenyl-(C₃-C₄)-alkenylthio, (C₁-C₆)-alkyl-(C₃-C₈)-cycloalkylthio, (C₂-C₆)-alkenyl-(C₃-Cₛ)-cycloalkylthio, (C₂-C₆)-alkinyl-(C₃-C₈)-cydoalkylthio, (C₁-C₆)-alkyl-( C₄-C₈)-cycloalkenylthio, (C₂-C₆)-alkenyl-(C₄-C₈)-cycloalkenylthio, (C₁-C₆)-alkylsulfinyl, (C₃-C₆)-alkenylsulfinyl, (C₃-C₆)-alkinylsulfinyl, (C₁-C₆)-haloalkylsulfinyl, (C₃-C₆)-haloalkenylsulfinyl, (C₃-C₆)-haloalkinylsulfinyl, (C₃-C₈)-cycloalkylsulfinyl, (C₄-C₈)-cycloalkenylsulfinyl, (C₃-C₈)-halocycloalkylsulfinyl, (C₄-C₈)-halocycloalkenylsulfinyl, (C₃-c₈)-cycloalkyl-(C₁-C₄)-alkylsulfinyl, (C₄-C₈)-cycloalkenyl-(C₁-C₄)-alkylsulfinyl, (C₃-C₈)-cydoalkyl-(C₃-C₄)-alkenylsulfinyl, (C₄-C₈)-cycloalkenyl-(C₃-C₄)-alkenylsulfinyl, (C₁-C₆)-alkyl-(C₃-C₈)-cycloalkyl-sulfinyl, (C₂-C₆)-alkenyl-(C₃-C₈)-cydoalkylsulfinyl, (C₂-C₆)-alkinyl-(C₃-C₈)-cycloalkylsulfinyl, (C₁-C₆)-alkyl-(C₄-C₈)-cycloalkenylsulfinyl, (C₂-C₆)-alkenyl-(C₄-C₈)-cycloalkenylsulfinyl, (C₁-C₆)-alkylsulfonyl, (C₃-C₆)-alkenylsulfonyl, (C₃-C₆)-alkinylsulfonyl, (C₁-C₆)-haloalkylsulfonyl, (C₃-C₆)-haloalkenylsulfonyl, (C₃-C₆)-haloalkinylsulfonyl, (C₃-C₈)-cycloalkylsulfonyl, (C₄-C₈)-cycloalkenylsulfonyl, (C₃-C₈)-halocycloalkylsulfonyl, (C₄-C₈)-halocydoalkenylsulfonyl, (C₃-C₈-cycloalkyl-(C₁-C₄)-alkylsulfonyl, (C₄-C₈)-cycloalkenyl-(C₁-C₄)-alkylsulfonyl, (C₃-C₈)-cycloalkyl-(C₃-C₄)-alkenylsulfonyl, (C₄-C₈)-cycloalkenyl-(C₃-C₄)-alkenylsulfonyl, (C₁-C₆)-alkyl-(C₃-C₈)-cycloalkylsulfonyl, (C₂-C₆)-alkenyl-(C₃-C₈)-cycloalkylsulfonyl, (C₂-C₆)-alkinyl-(C₃-C₈)-cycₗoalkylsulfonyl, (C₁-C₆)-alkyl-(C₄-C₈)-cycloalkenylsulfonyl, (C₂-C₆)-alkenyl-(C₄-C₈)-cycloalkenylsulfonyl, (C₁-C₆)-dialkyl-amino, (C₁-C₆)-alkylamino, (C₃-C₆)-alkenylamino, (C₃-C₆)-alkinylamino, (C₁-C₆)-haloalkylamino, (C₃-C₆)-haloalkenylamino, (C₃-C₆)-haloalkinyl-amino, (C₃-C₈)-cycloalkylamino, (C₄-C₈)-cydoalkenylamino, (C₃-C₈)-halocycloalkamino, (C₄-C₈)-halocycloalkenylamino, (C₃-C₈)-cycloalkyl-(C₁-C₄)-alkylamino, (C₄-C₈)-cycloalkenyl-(C₁-C₄)-alkylamino, (C₃-C₈)-cycloalkyl-(C₃-C₄)-alkenylamino, (C₄-C₈)-cycloalkenyl-(C₃-C₄)-alkenyl-amino, (C₁-C₆)-alkyl-(C₃-C₈)-cycloalkylamino, (C₂-C₆)-alkenyl-(C₃-C₈)-cycloalkylamino, (C₂-C₆)-alkinyl-(C₃-C₈)-cycloalkylamino, (C₁-C₆)-alkyl-(C₄-C₈)-cycloalkenylamino, (C₂-C₆)-alkenyl-(C₄-C₈)-cycloalkenylamino, (C₁-C₆)-trialkylsilyl, aryl, aryloxy, arylthio, arylsulfinyl, arylsulfonyl, arylamino, aryl-(C₁-C₄)-alkoxy, aryl-(C₃-C₄)-alkenyloxy, aryl-(C₁-C₄)-alkylthio, aryl-(C₁-C₄)-alkylsulfinyl, aryl-(C₁-C₄)-alkylsulfonyl, aryl-(C₂-C₄)-alkenylthio, aryl-(C₂-C₄)-alkenylsulfinyl, aryl-(C₂-C₄)-alkenylsulfo-nyl, aryl-(C₁-C₄)-alkylamino, aryl-(C₃-C₄)-alkenylamino, aryl-(C₁-C₆)-dialkylsilyl, diaryl-(C₁-C₆)-alkylsilyl, triarylsilyl and 5- or 6-membered heterocyclyl, wherein the cyclic part of the last mentioned 14 residues optionally has one or more substituents selected from the group consisting of halogen, cyano, nitro, amino, hydroxyl, thio, (C₁-C₄)-alkyl, (C₁-C₄)-haloalkyl, (C₃-C₈)-cycloalkyl, (C₁-C₄)-alkoxy, (C₁-C₄)-haloalkoxy, (C₁-C₄)-alkylthio, (C₁-C₄)-haloalkylthio, (C₁-C₄)-alkylamino, (C₁-C₄)-haloalkylamino and (C₁-C₄)-alkanoyl, and, if R⁹ is aryl or heterocyclyl, (C₁-C₄)-alkyl or (C₁-C₄)-haloalkyl.

7. Compounds of formula (II) and salts thereof according to claim 2, wherein R¹ is SF₅, CHF₂, CF₂Cl or CF₃.

8. Compounds of formula (II) and salts thereof according to claim 2 or 7, wherein X is =CH, Y' is -O-, n is 0, R¹ is CF₃, R², R³ and R⁴' are hydrogen and R⁶ is (C₁-C₁₀)-alkyl, (C₃-C₈)-cycloalkyl, aryl, benzyl or heterocyclyl containing one to three nitrogen, oxygen and/or sulfur ring atoms wherein the mentioned residues are optionally substituted with one or more residues.

9. Compounds of formula (II) and salts thereof according to claim 2, 7 or 8, wherein the residues R^{4'} and R⁶ carry the residues R⁷ as substituents with the following meaning: R⁷ is identical or different R⁸ or two residues R⁷ form, together with the atoms to which they are bound, a 3- to 8-membered saturated or unsaturated ring system which optionally is substituted with one or more residues R⁸ and optionally comprises additional heteroatoms selected from the group consisting of O, N, S, SO and SO₂; and R⁸ has the meaning as defined in claim 6.

10. Method for the manufacture of the compounds of formula (I) according to claims 1, 3, 4, 5 and 6 comprising the steps: 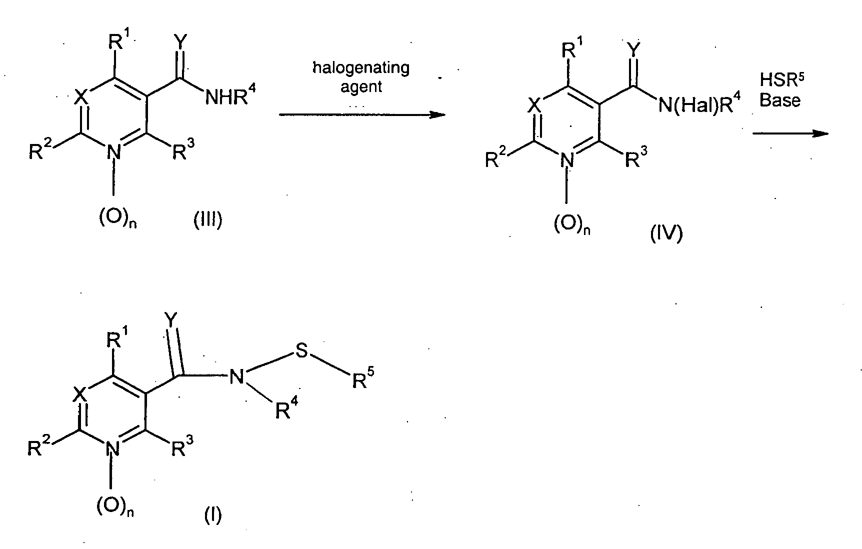
a) Reaction of a carboxamide of formula (II) with a halogenating agent to give the compound of formula (IV), and
b) Reaction of this compound with a thioether R⁵SH in the presence of a base to give the end products of formula (I), wherein in these formulae the residues R¹, R², R³, R⁴, R⁵, R⁶, X and Y as well as index n have the meaning as defined in claim 1 and Hal means halogen.

11. Method for the manufacture of compounds of formula (I) according to one or more of the claims 1, 3, 4, 5 and 6 comprising the reaction of an activated derivative of the carboxylic acid or thiocarboxylic acid of formula (VI) 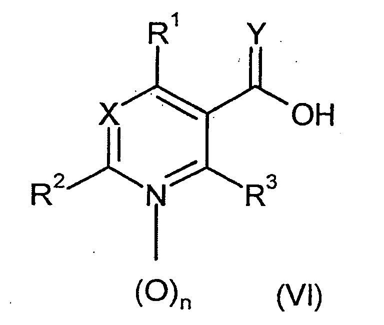 in the presence of a base together with a compound of formula (Vll), 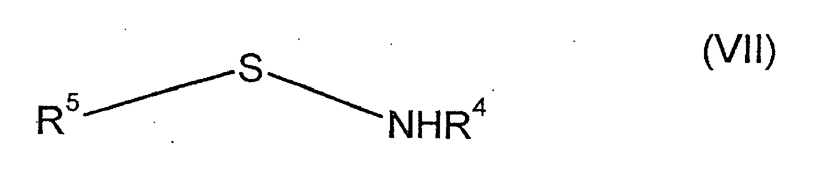 wherein in these formulae the residues R¹, R², R³, R⁴, R⁵, X and Y as well as n have the meaning as defined in claim 1.

12. Method for the manufacture of compounds of formula (la) according to claim 1 via thermal decomposition of the sulfimides of formula (VIII) 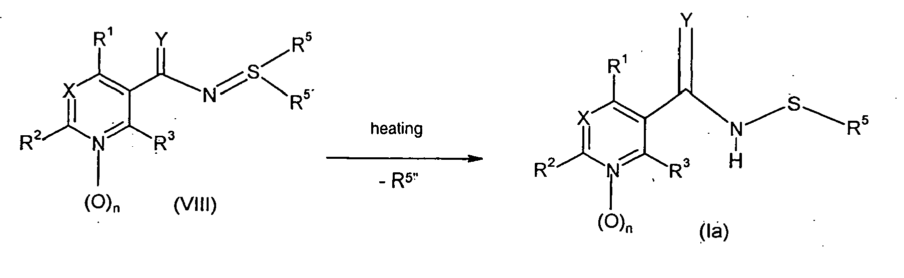 wherein the residues R¹, R², R³, R⁴, R⁵ , X and Y as well as the index n have the meaning as defined in claim 1, R⁵ is one of the residues defined as R⁵ in claim 1 which has a β-hydrogen atom, and R^{5"} is a residue which has one hydrogen atom less than the ethylenic unsaturated leaving-group corresponding to R⁵'.

13. Processes for the manufacture of compounds having the formula (II) according to one or more of the claims 2, 7, 8 and 9 comprising the reaction of the compounds of formula (Ib), 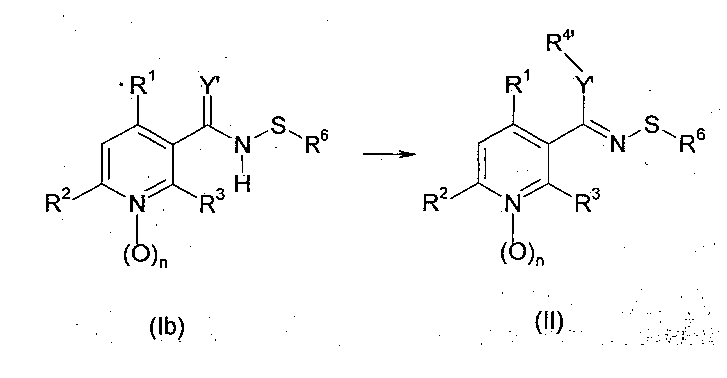 together with an alcohol of formula R^{4'}-OH in the presence of an azodicarboxylic acid diester as well as of a phosphine according to the scheme above to give compounds of formula (II), wherein R^{4'} has one of the meanings as defined in claim 2 excluding H and wherein R¹, R², R³, R⁶, X, Y' and n have the meaning as defined in claim 2.

14. Composition having insecticidal, acaricidal, ixodicidal, nematicidal, molluskicidal and/or fungicidal effect which contain at least one of the compounds of formula (I) and/or (II) or one of the salts thereof according to claim 1 and/or 2.

15. Use of the compounds of formula (I) and/or (II) or one of the salts thereof according to claim 1 and/or 2 for the preparation of a composition or an agent for controlling pest.

16. Use of the compounds of formula (I) and/or (II) or one of the salts thereof according to claim 1 and/or 2 for the preparation of a composition or an agent for the repelling or the banishment of pest.

17. Use of compounds of formula (I) and/or (II) or one of the salts thereof according to claim 1 and/or 2 for the preparation of a veterinary medicament.

18. Method for pest control, wherein the pests are directly or indirectly contacted with a compound of formula (I) or (II) or one of the salts thereof according to claim 1 or 2, with the exception of uses or methods practiced on the human or animal body.

19. Method for the repelling or the banishment of pest organisms, wherein one or more of the compounds of formula (I) and/or (II) or one of the salts thereof as according to claim 1 and/or 2 is deployed at a place, from which the pest organisms should be repelled or banned, with the exception of uses or methods practiced on the human or animal body.

20. Method for the manufacture of sulfimides of formula (VIII) via reaction of a thiohydroxamic acid having the general formula (Ia) in the presence of a compound R^{5'}-Z and a base according to the following scheme: 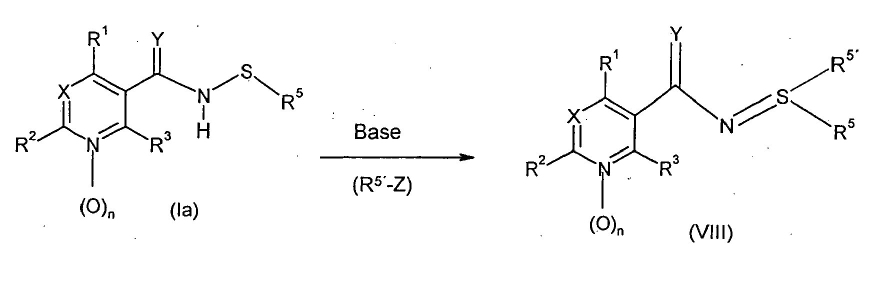 wherein the residues R¹, R², R³, R⁵, X and Y as well as the index n have the meaning as defined in claim 1, R⁵' is independent of R⁵ one of the groups as defined as R⁵ in claim 1, and Z is a leaving-group.

## Revendications

1. Composés de la formule (I) et leurs sels, 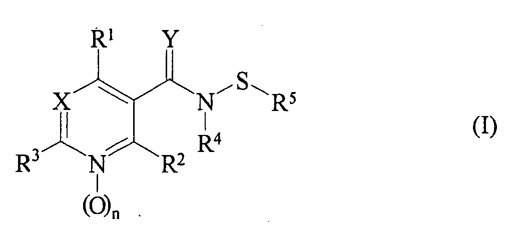 dans laquelle les symboles et les indices ont les significations suivantes :
X est =CH- ou =N- ;
Y est =O ou =S ;
n vaut 0 ou 1 ;
R¹ est alkyle en C₁ à C₆, haloalkyle en C₁ à C₆, -S(halogène)₅ ou halogène, un ou deux groupes CH₂ pouvant être remplacés par -O- ou -S- ou -N-alkyle en C₁ à C₆, à la condition que les hétéroatomes ne soient pas voisins ;
R², R³ sont indépendamment l'un de l'autre hydrogène, alkyle en C₁ à C₆, haloalkyle en C₁ à C₆ ou halogène, un ou deux groupes CH₂ pouvant être remplacés par -O- ou -S- ou N-alkyle en C₁ à C₆, à la condition que les hétéroatomes ne soient pas voisins ;
R⁴ est hydrogène, alkyle en C₁ à C₁₀, cycloalkyle en C₃ à C₁₀, alcényle en C₃ à C₁₀, alcynyle en C₃ à C₁₀, aryle en C₆ à C₁₄, hétérocyclyle en C₃ à C₁₀ ou alcanoyle en C₁ à C₁₀, les résidus précités pouvant être facultativement uni- ou plurisubstitués ;
R⁵ est hydrogène, alkyle en C₁ à C₁₀, alcényle en C₃ à C₁₀, alcynyle en C₃ à C₁₀, cycloalkyle en C₃ à C₈, cycloalcényle en C₄ à C₈, cycloalcynyle en C₈ à C₁₀, aryle ou hétérocyclyle, les résidus précités pouvant être facultativement uni- ou plurisubstitués ;
à l'exclusion des composés de la formule (I) dans lesquels X est =CH-et R⁵ est un alkyle en C₁ à C₁₀ le cas échéant substitué.

2. Composés de la formule (II) et leurs sels, 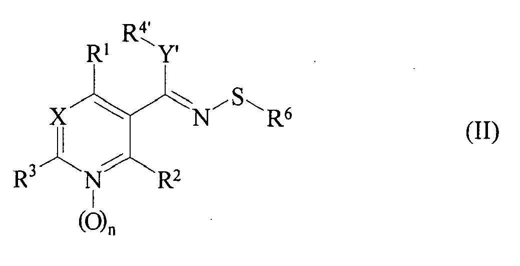 dans laquelle les symboles et les indices ont les significations suivantes :
X est =CH- ou =N- ;
Y' est -O- ou -S- ;
N vaut 0 ou 1 ;
R¹ est alkyle en C₁ à C₆, haloalkyle en C₁ à C₆, -S(halogène)₅ ou halogène, un ou deux groupes CH₂ pouvant être remplacés par -O- ou -S- ou -N-alkyle en C₁ à C₆, à la condition que les hétéroatomes ne soient pas voisins ;
R², R³ sont indépendamment l'un de l'autre hydrogène, alkyle en C₁ à C₆, haloalkyle en C₁ à C₆ ou halogène, un ou deux groupes CH₂ pouvant être remplacés par -O- ou -S- ou N-alkyle en C₁ à C₆, à la condition que les hétéroatomes ne soient pas voisins ;
R⁴' est hydrogène, alkyle en C₁ à C₁₀, cycloalkyle en C₃ à C₁₀, alcényle en C₃ à C₁₀, alcynyle en C₃ à C₁₀, aryle en C₆ à C₁₄ ou hétérocyclyle en C₃ à C₁₀, les résidus précités pouvant être facultativement uni- ou plurisubstitués ; et
R⁶ est hydrogène, alkyle en C₁ à C₁₀, alcényle en C₃ à C₁₀, alcynyle en C₃ à C₁₀, cycloalkyle en C₃ à C₈, cycloalcényle en C₄ à C₈, cycloalcynyle en C₈ à C₁₀, aryle ou hétérocyclyle, les résidus précités pouvant être facultativement uni- ou plurisubstitués.

3. Composés de la formule (I) et leurs sels selon la revendication 1, dans lesquels R¹ est SF₅, CHF₂, CF₂Cl ou CF₃.

4. Composés de la formule (I) et leurs sels selon la revendication 1 ou 3, dans lesquels R² et R³ sont hydrogène.

5. Composés de la formule (I) et leurs sels selon l'un ou plusieurs des revendications 1, 3 ou 4, dans lesquels X =CH- est, Y est =O, n vaut 0, R¹ est CF₃, R², R³ et R⁴ sont hydrogène et R⁵ est alkyle en C₁ à C₁₀, alcényle en C₂ à C₁₀, alcynyle en C₂ à C₁₀, cycloalkyle en C₃ à C₈, cycloalcényle en C₄ à C₈, cycloalcynyle en C₈ à C₁₀, aryle ou hétérocyclyle, les résidus précités pouvant être facultativement uni- ou plurisubstitués.

6. Composés de la formule (I) et leurs sels selon l'un ou plusieurs des revendications 1, 3, 4 ou 5, dans lesquels R⁵ est un résidu de la formule IIa, 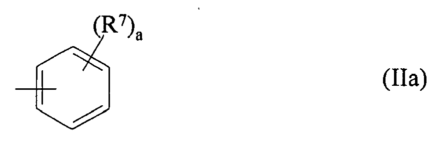 dans laquelle les symboles et les indices ont les significations suivantes :
a vaut 0, 1, 2, 3 ou 4 ;
R⁷ est identique ou différent et représente R⁸ ou bien deux résidus R⁷ pris ensemble avec les atomes auxquels ils sont rattachés forment un système cyclique de trois à huit termes, saturé ou insaturé, le cas échéant substitué par un ou plusieurs résidus R⁸ et contenant également le cas échéant d'autres hétéroatomes du groupe O, N, S, SO et SO₂;
R⁸ est identique ou différent et représente R⁹, R¹⁰, -C(W)R⁹, -C(=NOR⁹)R⁹, -C(=NNR⁹₂)R⁹, -C(=W)OR⁹, -C(=W)NR⁹₂, -OC(=W)R⁹, -OC(=W)OR⁹, NR⁹C(=W)R⁹, N[C(=W)R⁹]₂, -NR⁹C(=W)OR⁹, -C(=W)NR⁹-NR⁹₂, -C(=W)NR⁹, NR⁹[C(=W)R⁹], -NR⁹-C(=W)NR⁹₂, NR⁹-NR⁹C(=W)R⁹, -NR⁹-N[C(=W)R⁹h, -N[(C-=W)R⁹]-NR⁹₂, -NR⁹-N[(C=W)WR⁹], -NR⁹, [(C=W)NR⁹₂], -NR⁹(C=NR⁹)R⁹, -NR⁹(C=NR⁹)NR⁹₂, -O-NR⁹₂, -O-NR⁹ (C=W)R⁹, -SO₂NR⁹₂, -NR⁹SO₂R⁹, -SO₂OR⁹, -OSO₂R⁹, -OR⁹, -NR⁹₂, -SR⁹, -SiR⁹₃, -PR⁹₂, -P(=W)R⁹₂, -SOR⁹, -SO₂R⁹, -PW₂R⁹₂, -PW₃R⁹₂, ou bien deux résidus R⁸ pris ensemble sont (=W), (=N-R⁹), (=CR₂⁹), (=CHR⁹) ou (=CH₂) ;
W est =O ou =S ;
R⁹ est identique ou différent et représente un alkyle en C₁ à C₆, alcényle en C₂ à C₆, alcynyle en C₂ à C₆, cycloalkyle en C₃ à C₈, cycloalcényle en C₄ à C₈, (cycloalkyle en C₃ à C₈)-(alkyle en C₁ à C₄), (cycloalcényle en C₄ à C₈)-(alkyle en C₁ à C₄), (cycloalkyle en C₃ à C₈)-(alcényle en C₂ à C₄), (cycloalcényle en C₄ à C₈)-(alcényle en C₂ à C₄), (alkyle en C₁ à C₆)-(cycloalkyle en C₃ à C₈), (alcényle en C₂ à C₆)-(cycloalkyle en C₃ à C₈), (alcynyle en C₂ à C₆)-(cycloalkyle en C₃ à C₈), (alkyle en C₁ à C₆)-(cycloalcényle en C₄ à C₈), (alcényle en C₂ à C₆)-(cycloalcényle en C₄ à C₈), aryle, hétérocyclyle ; les résidus précités étant le cas échéant substitués par un ou plusieurs résidus R¹⁰ et, le cas échéant, deux résidus R⁹ pris ensemble forment un système cyclique ;
R¹⁰ est identique ou différent et représente un halogène, cyano, nitro, hydroxy, thio, amino, formyle, alcanoyle en C₁ à C₆, alcoxy en C₁ à C₆, alcényloxy en C₃ à C₆, alcynyloxy en C₃ à C₆, haloalkyloxy en C₁ à C₆, haloalcényloxy en C₃ à C₆, haloalcynyloxy en C₃ à C₆, cycloalcoxy en C₃ à C₈, cycloalcényloxy en C₄ à C₈, halocycloalcoxy en C₃ à C₈, halocycloalcényloxy en C₄ à C₈, (cycloalkyle en C₃ à C₈)-(alcoxy en C₁ à C₄), (cycloalcényle en C₄ à C₈)-(alcoxy en C₁ à C₄), (cycloalkyle en C₃ à C₈)-(alcényloxy en C₂ à C₄), (cycloalcényle en C₄ à C₈)-(alcényloxy en C₁ à C₄), (alkyle en C₁ à C₆)-(cycloalcoxy en C₃ à C₈), (alcényle en C₂ à C₆)-(cycloalcoxy en C₃ à C₈), (alcynyle en C₂ à C₆)-(cycloalcoxy en C₃ à C₈), (alkyle en C₁ à C₆)-(cycloalcényloxy en C₄ à C₈), (alcényle en C₂ à C₆)-(cycloalcényloxy en C₄ à C₈), (alcoxy en C₁ à C₄)-(alcoxy en C₁ à C₆), (alcoxy en C₁ à C₄)-(alcényloxy en C₃ à C₆), carbamoyle, mono- ou dialkylcarbamoyle en C₁ à C₆, mono- ou dihaloalkylcarbamoyle en C₁ à C₆, mono- ou dicycloalkylcarbamoyle en C₃ à C₈, alcoxycarbonyle en C₁ à C₆, cycloalcoxycarbonyle en C₃ à C₈, alcanoyloxy en C₁ à C₆, cycloalcanoyloxy en C₃ à C₈, haloalcoxycarbonyle en C₁ à C₆, haloalcanoyloxy en C₁ à C₆, alcanoylamino en C₁ à C₆, haloalcanoylamino en C₁ à C₆, alcénoylamino en C₂ à C₆, cycloalcanoylamino en C₃ à C₈, cycloalkyle en C₃ à C₈, alcanoylamino en C₁ à C₄, alkylthio en C₁ à C₆, alcénylthio en C₃ à C₆, alcynylthio en C₃ à C₆, haloalkylthio en C₁ à C₆, haloalcénylthio en C₃ à C₆, haloalcynylthio en C₃ à C₆, cycloalkylthio en C₃ à C₈, cycloalcénylthio en C₄ à C₈, halocycloalkylthio en C₃ à C₈, halocycloalcénylthio en C₄ à C₈, (cycloalkyle en C₃ à C₈)-(alkylthio en C₁ à C₄), (cycloalcényle en C₄ à C₈)-(alkylthio en C₁ à C₄), (cycloalkyle en C₃ à C₈)-(alcénylthio en C₃ à C₄), (cycloalcényle en C₄ à C₈)-(alcénylthio en C₃ à C₄), (alkyle en C₁ à C₆)-(cycloalkylthio en C₃ à C₈), (alcényle en C₂ à C₆)-(cycloalkylthio en C₃ à C₈), (alcynyle en C₂ à C₆)-(cycloalkylthio en C₃ à C₈), (alkyle en C₁ à C₆)-(cycloalcénylthio en C₄ à C₈), (alcényle en C₂ à C₆)-(cycloalcénylthio en C₄ à C₈), alkylsulfinyle en C₁ à C_{6,} alcénylsulfinyle en C₃ à C₆, alcynylsulfinyle en C₃ à C₆, haloalkylsulfinyle en C₁ à C₆, haloalcénylsulfinyle en C₃ à C₆, haloalcynylsulfinyle en C₃ à C₆, cycloalkylsulfinyle en C₃ à C₈, cycloalcénylsulfinyle en C₄ à C₈, halocycloalkylsulfinyle en C₃ à C₈, halocycloalcénylsulfinyle en C₄ à C₈, (cycloalkyle en C₃ à C₈)-(alkylsulfinyle en C₁ à C₄), (cycloalcényle en C₄ à C₈)-(alkylsulfinyle en C₁ à C₄), (cycloalkyle en C₃ à C₈)-(alcénylsulfinyle en C₃ à C₄), (cycloalcényle en C₄ à C₈)-(alcénylsulfinyle en C₃ à C₄), (alkyle en C₁ à C₆)-(cycloalkylsulfinyle en C₃ à C₈), (alcényle en C₂ à C₆)-(cycloalkylsulfinyle en C₃ à C₈), (alcynyle en C₂ à C₆)-(cycloalkylsulfinyle en C₃ à C₈), (alkyle en C₁ à C₆)-(cycloalcénylsulfinyle en C₄ à C₈), (alcényle en C₂ à C₆)-(cycloalcénylsulfinyle en C₄ à C₈), alkylsulfonyle en C₁ à C₆, alcénylsulfonyle en C₃ à C₆, alcynylsulfonyle en C₃ à C₆, haloalkylsulfonyle en C₁ à C₆, haloalcénylsulfonyle en C₃ à C₆, haloalcynylsulfonyle en C₃ à C₆, cycloalkylsulfonyle en C₃ à C₈, cycloalcénylsulfonyle en C₄ à C₈, halocycloalkylsulfonyle en C₃ à C₈, halocycloalcénylsulfonyle en C₄ à C₈, (cycloalkyle en C₃ à C₈)-(alkylsulfonyle en C₁ à C₄), (cycloalcényle en C₄ à C₈)-(alkylsulfonyle en C₁ à C₄), (cycloalkyle en C₃ à C₈)-(alcénylsulfonyle en C₃ à C₄), (cycloalcényle en C₄ à C₈)-(alcénylsulfonyle en C₃ à C₄), (alkyle en C₁à C₆)-(cycloalkylsulfonyle en C₃ à C₈), (alcényle en C₂ à C₆)-(cycloalkyle en C₃ à C₈)sulfonyle, (alcynyle en C₂ à C₆)-(cycloalkylsulfonyle en C₃ à C₈), (alkyle en C₁ à C₆)-(cycloalcénylsulfonyle en C₄ à C₈), (alcényle en C₂ à C₆)-(cycloalcénylsulfonyle en C₄ à C₈), dialkylamino en C₁ à C₆, alkylamino en C₁ à C₆, alcénylamino en C₃ à C₆, alcynylamino en C₃ à C₆, haloalkylamino en C₁ à C₆, haloalcénylamino en C₃ à C₆, haloalcynylamino en C₃ à C₆, cycloalkylamino en C₃ à C₈, cycloalcénylamino en C₄ à C₈, halocycloalkylamino en C₃ à C₈, halocycloalcénylamino en C₄ à C₈, (cycloalkyle en C₃ à C₈)-(alkylamino en C₁ à C₄), (cycloalcényle en C₄ à C₈)-(alkylamino en C₁ à C₄), (cycloalkyle en C₃ à C₈)-(alcénylamino en C₃ à C₄), (cycloalcényle en C₄ à C₈)-(alcénylamino en C₃ à C₄), (alkyle en C₁ à C₆)-(cycloalkylamino en C₃ à C₈), (alcényle en C₂ à C₆)-(cycloalkylamino en C₃ à C₈), (alcynyle en C₂ à C₆)-(cycloalkylamino en C₃ à C₈), (alkyle en C₁) à C₆)-(cycloalcénylamino en C₄ à C₈), (alcényle en C₂ à C₆)-(cycloalcénylamino en C₄ à C₈), trialkylsilyle en C₁ à C₆, aryle, aryloxy, arylthio, arylsulfinyle, arylsulfonyle, arylamino, aryl(alcoxy en C₁ à C₄), aryl(alcényloxy en C₃ à C₄), aryl(alkylthio en C₁ à C₄), aryl(alkylsulfinyle en C₁ à C₄), aryl-(alkylsulfonyle en C₁ à C₄), aryl(alcénylthio en C₂ à C₄), aryl(alcénylsulfinyle en C₂ à C₄), aryl(alcénylsulfonyle en C₂ à C₄), aryl(alkylamino en C₁ à C₄), aryl(alcénylamino en C₃ à C₄), aryl(dialkylsilyle en C₁ à C₆), diaryl(alkylsilyle en C₁ à C₆ à, triarylsilyle et hétérocyclyle de 5 ou 6 termes, la partie cyclique des quatorze derniers résidus cités étant le cas échéant substituée par un ou plusieurs résidus du groupe comprenant un halogène, cyano, nitro, amino, hydroxy, thio, alkyle en C₁ à C₄, haloalkyle en C₁ à C₄, cycloalkyle en C₃ à C₈, alcoxy en C₁ à C₄, haloalcoxy en C₁ à C₄, alkylthio en C₁ à C₄, haloalkylthio en C₁ à C₄, alkylamino en C₁ à C₄, haloalkylamino en C₁ à C₄ et alcanoyle en C₁ à C₄, et, lorsque R⁹ est un aryle ou un hétérocyclyle, par un alkyle en C₁ à C₄ ou un haloalkyle en C₁ à C₄.

7. Composés de la formule (II) et leurs sels selon la revendication 2, dans lesquels R¹ est SF₅, CHF₂, CF₂Cl ou CF₃.

8. Composés de la formule (II) et leurs sels selon la revendication 2 ou 7, dans lesquels X est =CH-, Y' est -O-, n vaut 0, R¹ est CF₃, R², R³ et R⁴' sont hydrogène et R⁶ est alkyle en C₁ à C₁₀, cycloalkyle en C₃ à C₈, aryle, benzyle ou hétérocyclyle avec au total un à trois atomes d'azote, d'oxygène et/ou de soufre dans le noyau cyclique, les résidus précités pouvant être facultativement uni- ou plurisubstitués.

9. Composés de la formule (II) et leurs sels selon la revendication 2, 7 ou 8, dans lesquels, en tant que substituants sur les résidus R^{4'} et R⁶ les groupes R⁷ sont présents avec la signification suivante : R⁷ identique ou différent, est R⁸ ou bien deux résidus R⁷ pris ensemble avec les atomes auxquels ils sont rattachés forment un système cyclique de trois à huit termes, saturé ou insaturé, le cas échéant substitué par un ou plusieurs résidus R⁸ et contenant également le cas échéant d'autres hétéroatomes du groupe O, N, S, SO et SO₂ ; et R⁸ a la signification définie dans la revendication 6.

10. Procédé de préparation de composés de la formule (I) selon l'une ou plusieurs des revendications 1, 3, 4, 5 et 6, comprenant les étapes consistant à : 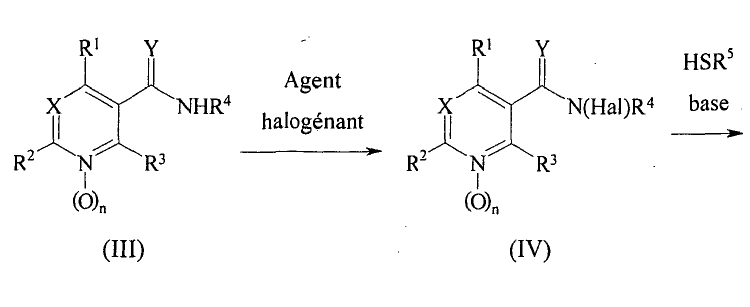 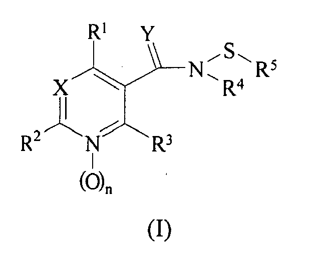
a) Faire réagir un amide d'acide carboxylique de la formule (III) avec un agent halogénant pour obtenir un composé de la formule (IV), et
b) Faire réagir ce composé avec un thioéther R⁵SH en présence d'une base pour obtenir les produits finaux de la formule (I), dans lesquels, dans ces formules, les résidus R¹, R², R³, R⁴, R⁵, X et Y ainsi que l'indice n ont les significations indiquées dans la revendication 1 et Hal désigne un halogène.

11. Procédé de préparation de composés de la formule (I) selon l'un ou plusieurs des revendications 1, 3, 4, 5 et 6, comprenant la réaction d'un dérivé activé de l'acide carboxylique ou thiocarboxylique de la formule (VI) 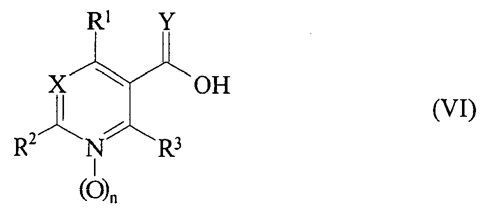 en présence d'une base avec un composé de la formule (VII), 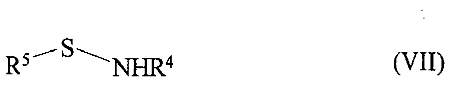 dans lesquels, dans ces formules, les résidus R¹, R², R³, R⁴, R⁵, X et Y ainsi que l'indice n ont les significations indiquées dans la revendication 1.

12. Procédé de préparation de composés de la formule (Ia) selon la revendication 1 par décomposition thermique des sulfimides de la formule (VIII) 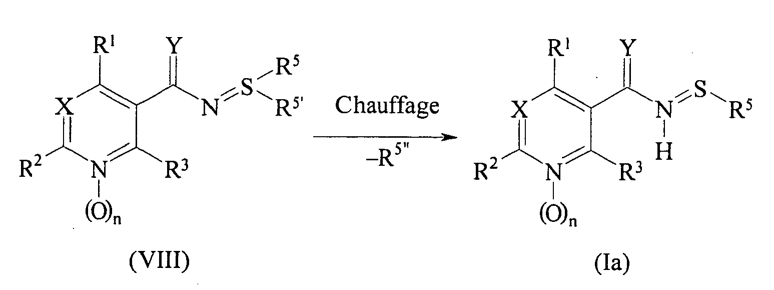 où les résidus R¹, R², R³, R⁵, X et Y ainsi que l'indice n ont les significations indiquées dans la revendication 1, R⁵' représente un des groupes définis dans la revendication 1 pour R⁵ qui présente un atome d'hydrogène β, et R^{5"} représente le groupe partant éthyléniquement insaturé correspondant à R^{5'} diminué de la teneur d'un atome d'hydrogène.

13. Procédé de préparation de composés de la formule (II) selon l'un ou plusieurs des revendications 2, 7, 8 et 9, comprenant la réaction des composés de la formule (Ib), 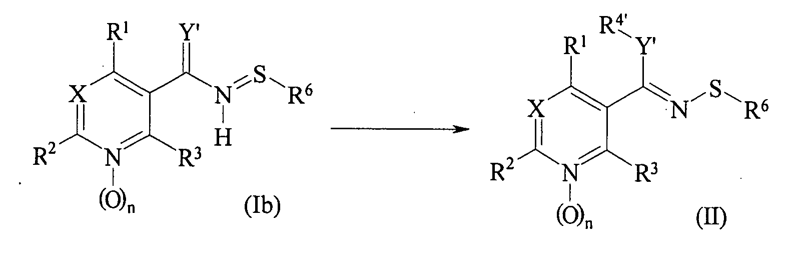 avec un alcool R ^{4'}-OH en présence d'un diester d'acide azodicarboxylique ainsi que d'une phosphine suivant le schéma ci-dessus pour obtenir les composés de la formule (II), dans lesquels R⁴' a l'une des significations définies dans la revendication 2, à l'exception de H, et R¹, R², R³, R⁶, X, Y' et n ont l'une des significations définies dans la revendication 2.

14. Agent ayant une activité insecticide, acaricide, ixodicide, nématicide, molluscicide et/ou fongicide, **caractérisé en ce qu'**il contient au moins un composé de la formule (I) et/ou (II) ou un de ses sels selon la revendication 1 et/ou la revendication 2.

15. Utilisation des composés de la formule (I) et/ou (II) ou de leurs sels selon la revendication 1 et/ou 2 pour préparer une composition ou un agent destiné(e) à lutter contre des animaux nuisibles.

16. Utilisation des composés de la formule (I) et/ou (II) ou de leurs sels selon la revendication 1 et/ou 2 pour préparer une composition ou un agent destiné(e) à lutter contre ou chasser des animaux nuisibles et/ou commensaux.

17. Utilisation des composés de la formule (I) et/ou (II) ou de leurs sels selon la revendication 1 et/ou 2 pour préparer un médicament vétérinaire.

18. Procédé de lutte contre des animaux nuisibles, dans lequel les animaux nuisibles sont mis en contact direct ou indirect avec un composé de la formule (I) ou (II) ou ses sels selon la revendication 1 ou 2, à l'exception des utilisations ou des procédés concernant le corps humain ou animal.

19. Procédé pour lutter contre ou chasser des organismes nuisibles, dans lequel un ou plusieurs composés de la formule (I) et/ou (II) ou leurs sels selon la revendication 1 et/ou 2 sont appliqués à l'endroit dont les organismes nuisibles doivent être tenus éloignés ou chassés, à l'exception des utilisations ou des procédés concernant le corps humain ou animal.

20. Procédé de préparation de sulfimides de la formule (VIII) en faisant réagir un acide thiohydroxamique de la formule générale (Ia) en présence d'un composé R^{5'}-Z et d'une base suivant le schéma : 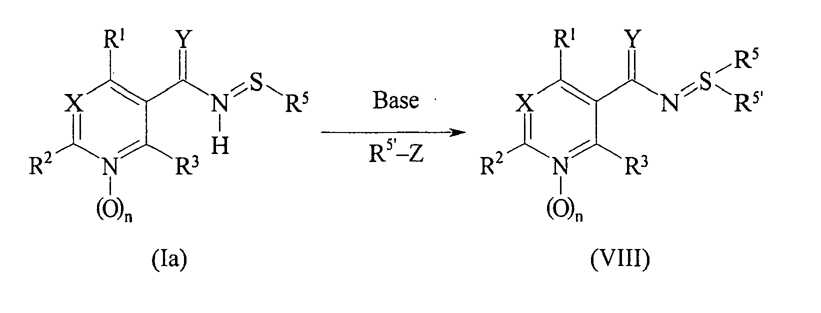 où les résidus R¹, R², R³, R⁵, X et Y ainsi que l'indice n ont les significations définies dans la revendication 1, R^{5'} représente indépendamment de R⁵ un des groupes définis dans la revendication 1 pour R⁵, et Z est un groupe partant.
